# EUROPÄISCHE PATENTSCHRIFT

(11) **EP 0 494 702 B1**
(45) Veröffentlichungstag und Bekanntmachung des Hinweises auf die Patenterteilung: **13.10.1999**
(21) Anmeldenummer: 92105735.2
(22) Anmeldetag: 06.03.1987
(51) Int. Cl.: C12N 15/54, C12N 9/10, C07K 14/745, C12P 21/02, C12Q 1/56, A61K 38/36, C12P 21/08, G01N 33/573

(54) **Gentechnische Herstellung von Faktor XIIIa**
Production of factor XIIIa by gene technology
Production de facteur XIIIa par technologie génétique

(30) Priorität: 12.03.1986 DE 3608280; 26.06.1986 DE 3621371
(43) Veröffentlichungstag der Anmeldung: 15.07.1992
(62) Teilanmeldung aus: 87103222.3
(73) Patentinhaber: BEHRINGWERKE Aktiengesellschaft, 35001 Marburg (DE)
(72) Erfinder: Grundmann, Ulrich, Dr., W-3551 Lahntal (DE); Amann, Egon, Dr., Tokyo 154 (JP); Zettlmeissl, Gerd, Dr., W-3551 Lahntal (DE)

(56) Entgegenhaltungen:
- EP-A- 0 018 561
- BIOCHEMISTRY, Band 13, Nr. 4, 1974, Washington, DC (US); T. TAKAGI et al., Seiten 750-756
- GENE, Band 40, 1985, Elsevier Science Publ., Amsterdam (NL); E. AMANN et al., Seiten 183-190
- BIOCHEMICAL JOURNAL, Band 135, 1973, London (GB); J.J. HOLBROOK et al., Seiten 901-903
- THE JOURNAL OF BIOLOGICAL CHEMISTRY Bd. 248, Nr. 4, 1973, Seiten 1395 - 1407 SCHWARTZ, M.L. ET AL. 'Human factor XIII from plasma and platelets'
- J. BIOCHEM. Bd. 78, 1975, Seiten 1247 - 1266 NAKAMURA, S. ET AL. 'On the activation of bovine plasma factor XIII'

## Beschreibung

Der Gerinnungsfaktor XIII ist das Endglied der "Gerinnungskaskade" beim natürlichen Prozess der Blutgerinnung bei den Wirbeltieren. Die enzymatisch aktive Form von Faktor XIII, Faktor XIII a, auch "aktivierter Fibrin-stabilisierender Faktor", "Fibrinoligase" oder "Plasma-Transglutaminase", im folgenden auch "F XIII a" genannt, katalysiert die Fusion von Fibrin-Einheiten in bereits existierenden Gerinnungspfropfen durch intramolekulare Vernetzung (Lorand et al., Methods in Enzymology 80 (1981), 333-341; Curtis et al., Annals New York Academy of Sciences 1983, 567-576). Das Molekulargewicht von Faktor XIII aus Plasma beträgt ca. 300 kD (Loewy et al., J. Biol. Chem. 236 (1961) 2634). Das Molekulargewicht der aktiven Untereinheit F XIII a beträgt ca. 80 kD (Bohn und Schwick, Arzneimittelforschung 21 (1971) 1432). Während der Aktivierung von Faktor XIII wird durch Thrombin ein ca. 4 kD großes Peptid aus der Vorstufe abgespalten, dessen Sequenz von 36 Aminosäuren bekannt ist (Takagi und Doolittle, Biochemistry 13 (1974) 750-756). Darüber hinaus ist eine vier Aminosäuren umfassende Sequenz bekannt (Holbrook et al., Biochem. J. 135 (1973) 901-903).

Die in Schwartz, M.L. et al. (1973), The Journal of Biological Chemistry 248, No. 4, 1395-1407 und Nakamura, S. et al. (1975), J. Biochem. 78, 1247-1266 beschriebenen F XIII Proteine besitzen eine andere Aminosäuresequenz.

Die Erfindung betrifft ein Verfahren zur gentechnischen Herstellung von F XIII a sowie hochreinen F XIII a mit der Aminosäuresequenz gemäß nachfolgender Tab. 3.

Die Zeichnungen, deren Nummern mit denen der Beispiele übereinstimmen, veranschaulichen die Erfindung:

Fig. 1 zeigt die für F XIIIa codierende CDNA (wobei der codierende Bereich schraffiert ist) und darunter die DNA-Bereiche der isolierten und charakterisierten Klone. Fig. 2 zeigt die Konstruktion des Expressionsplasmids pFXIII-13. Zur Verdeutlichung sind hier die Ausgangsplasmide pIC19H-12.1 und pIC19H-11.1 sowie die unmittelbar darunterangeordneten DNA-Fragmente durch Doppellinien dargestellt, ebenso das aus den Einzelstrang-Fragmenten konstruierte Produkt pFXIII-13.

Fig. 3 zeigt schematisch die Konstruktion des Plasmids pTrc97A, Fig. 3a diejenige von pFXIII-C4 aus pTrc97A und pFXIII-13, Fig. 3b schließlich die Konstruktion von pMB259 aus pFXIII-13 und den bekannten Plasmiden plC2OH und pBD2.

Fig. 4 zeigt schematisch die Konstruktion des Plasmids pMB240 aus pFXIII-13 und dem bekannten Plasmid pAAH5.

Fig. 5 zeigt die Konstruktion von pZET4 aus dem bekannten Plasmid pSV2dhfr und dem Plasmid pSVA STOP1, Fig. 5a die Konstruktion von pSVF13 aus pSVA STOPI und pFXIII-13, Fig. 5b die Konstruktion von pZF13 aus pZET4 und pFXIII-13 und schließlich Fig. 5c die Konstruktion von pHSF13 aus pSVF13 und dem bekannten Plasmid pSP6HS9.

Zur Konstruktion geeigneter Sonden wurde die Aminosäuresequenz von F XIIIa-Fragmenten ermittelt. Die entsprechenden Peptidfragmente wurden durch proteolytische oder Bromcyan-Spaltung gewonnen. Basierend auf der Kenntnis der Aminosäuresequenzen solcher Fragmente wurden zwei Oligonucleotidsonden synthetisiert, ein 20mer und ein 66mer.

Bei der 20mer-Sonde wurden alle theoretisch möglichen Codons für die Aminosäuresequenz Met-Met-Asp-Ile-Thr-Asp-Thr berücksichtigt, wobei im Falle der letzten Aminosäure die dritte Position im Codon entfiel. Die 20mer-Sonde ist somit 48-fach degeneriert, d.h. ein Gemisch aller 48 theoretisch möglichen codierenden Oligonucleotide der genannten Aminosäuresequenz (Tabelle 1; Anhang).

Die 66mer-Sonde wurde anhand der folgenden Aminosäuresequenz 
Tyr-Gly-Gln-Phe-Glu-Asp-Gly-Ile-Leu-Asp-Thr-
Cys-Leu-Tyr-Val-Met-Asp-Arg-Ala-Gln-Met-Asp
 aufgrund statistischer Daten (Lathe, J. Molec. Biol. 183 (1985) 1-12) ausgewählt (Tabelle 2, Anhang).

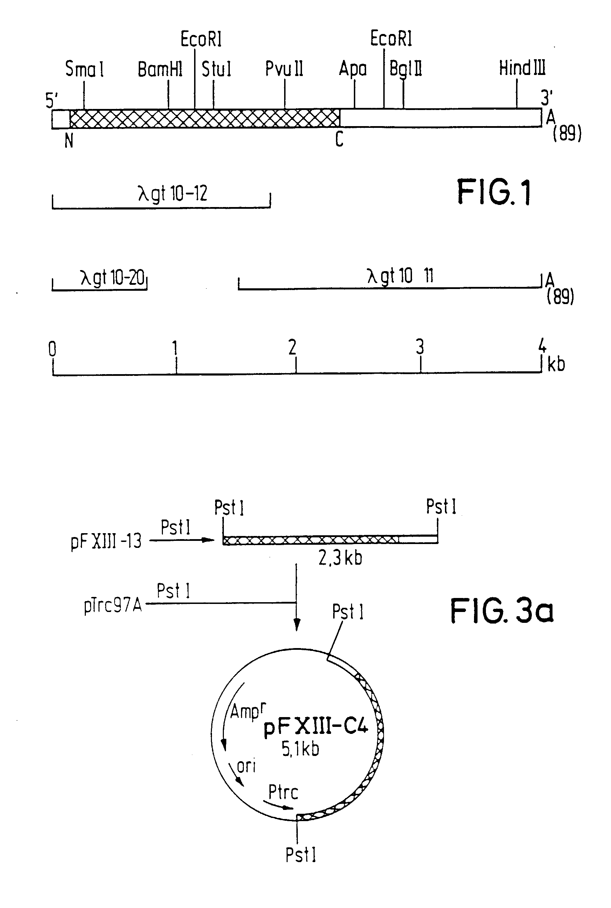

Mit diesen Sonden wurde eine cDNA-Bank einem "Screening" unterworfen. Die cDNA wurde aus mRNA von reifer, humaner Plazenta hergestellt, aus dieser die mRNA isoliert und daraus die cDNA hergestellt. Diese wurde mit EcoRI-Enden versehen und in die EcoRI-Schnittstelle des Phagenvektors λgt10 ligiert. Ein positiver Klon, λgt10-12, der mit der obengenannten Sonde identifiziert wurde, wurde weiteranalysiert (Fig. 1). Die Sequenzierung nach an sich bekannten Methoden ergab die DNA-Sequenz, die für F XIIIa codiert.

Durch "Rescreening" mit dieser DNA-Sequenz wurden aus der cDNA-Bank weitere Klone isoliert, die sowohl zum 5'- als auch zum 3'-Ende expandieren.

Die Fig. 1 zeigt die Restriktionskarte der DNA-Sequenz, die für F XIIIa codiert. "N" bezeichnet den N-Terminus, "C" den C-Terminus des codierenden Bereichs, "A₍₈₉₎" die Poly-(A)-Sequenz von 89 Basen. Diese Sequenz stellt die gesamte codierende Sequenz von F XIIIa dar. Die Tabelle 3 (Anhang) zeigt die ermittelte DNA-Sequenz (codierender Strang) und die daraus abgeleitete Aminosäure-Sequenz der klonierten cDNA-Fragmente aus λgt10-11 und λgt10-12. Die gesamte cDNA-hat eine Länge von 3905 Basenpaaren. Die von Takagi und Doolittle (a.a.O.) ermittelte N-terminale, 36 Aminosäuren umfassende Sequenz ist in der gefundenen Sequenz enthalten. Diese Sequenz ist in Tabelle 3 von Nucleotidposition 88-198 mit einer durchgehenden Linie markiert. Zusätzlich zu der von Takagi und Doolittle ermittelten Sequenz codiert die cDNA ein Valin in Nucleotidposition 187-189. Die von Holbrook et al. (a.a.O.) ermittelte vier Aminosäuren umfassende Sequenz Gly-Gln-Cys-Trp wird von der cDNA in Position 1021-1032 codiert. Diese Sequenz ist ebenfalls mit einer durchgehenden Linie markiert. Zusätzlich sind die Positionen der 20mer- und der 66mer-Oligonucleotidsonden durch unterbrochene Linien markiert. Die 20mer-Sonde hybridisiert von Position 1507-1526 und die 66mer-Sonde von Position 766-831.

Erfindungsgemäß kann die codierende cDNA dazu benutzt werden, modifizierte Gene herzustellen, die für Proteine mit veränderten biologischen Eigenschaften codieren. Hierzu können in an sich bekannter Weise Deletionen, Insertionen und Basenaustausche vorgenommen werden.

Durch die Auswahl des Wirtes kann weiterhin die Form der Modifikation des F XIIIa beeinflußt werden. So findet in Bakterien keine, in Hefezellen eine andere Glykosylierung als in höheren eukaryotischen Zellen statt.

In Kenntnis der Aminosäuresequenz von F XIIIa ist es möglich, nach konventionellen oder gentechnischen Methoden Aminosäure-Teilsequenzen herzustellen, die als Antigene zur Herstellung von polyklonalen oder monoklonalen Antikörpern dienen können. Solche Antikörper können nicht nur zu diagnostischen Zwecken, sondern auch zur Herstellung von Antikörpersäulen dienen, mit denen der F XIIIa aus Lösungen abgetrennt werden kann, die diesen Faktor neben anderen Proteinen enthalten.

Mit Hilfe der cDNA bzw. Teilen davon kann man auch einfach aus einer genomischen Bank den für F XIIIa codierenden genomischen Klon isolieren, mit dessen Hilfe nicht nur eine Expression in eukaryotischen Zellen möglich ist, sondern auch weitere diagnostische Aussagen getroffen werden können.

F XIIIa-Defizienzen können zu verschiedenen Krankheitsbildern führen, die zu einem großen Teil auf die Unfähigkeit zurückgeführt werden, aus den Vorläufern die aktive Form des Enzyms zu bilden. Die Kenntnis der cDNA von F XIIIa erlaubt nunmehr die Herstellung von Diagnostika, mit denen einfach festgestellt werden kann, ob genetische Veränderungen vorliegen.

Erfindungsgemäß kann man somit einen hochreinen Faktor XIIIa herstellen, bei dem nicht die Gefahr einer Verunreinigung durch beispielsweise Viren oder andere Proteine besteht. Die bisher bestehende Abhängigkeit von Human-Plasma oder Plazenten als Rohstoffquelle ist damit überwunden. Darüber hinaus eröffnet die Erfindung den Zugang für wertvolle Diagnostika und damit die Analyse genetisch bedingter F XIIIa-Defekte.

In den folgenden Beispielen wird die Erfindung näher erläutert. Prozentangaben, sofern sie sich nicht auf Mengen beziehen, betreffen das Gewicht, wenn keine anderen Angaben gemacht sind.

Soweit nicht im Text erläutert, wurden die folgenden Abkürzungen verwendet:
- EDTA =: Natrium-ethylendiamin-tetraacetat
- SDS =: Natrium-dodecylsulfat
- DTT =: Dithiothreitol
- BSA =: Rinderserumalbumin

### Beispiele:

### 1. Isolierung von RNA aus humaner Plazenta

RNA wurde aus reifer, humaner Plazenta (nach Chirgwin et al., Biochemistry 18 (1979) 5294-5299) gewonnen. Etwa 10 g Plazentagewebe wurden in flüssigem Stickstoff zermörsert, in 80 ml 4 Guanidinium-Thiocyanat mit 0,1 M Mercaptoethanol suspendiert und 90 sec. bei 20 000 rpm in einem Homogenisator (Ultraturrax) behandelt. Das Lysat wurde 15 min. bei 7 000 rpm zentrifugiert (Sorvall-GSA Rotor) und der Überstand mit 2 ml 1 M Essigsäure und 60 ml Ethanol abs. bei -20°C über Nacht gefällt. Nach Sedimentation bei 6 000 rpm und -10°C für 10 min. wurden die Nucleinsäuren in 40 ml 7,5 M Guanidinium-Hydrochlorid (pH 7,0) vollständig gelöst und mit einer Mischung aus 1 ml 1 M Essigsäure und 20 ml Ethanol abs. gefällt. Zur Abtrennung der DNA wurde die Fällung ein weiteres Mal mit jeweils halben Volumina wiederholt.
Die RNA wurde in 12 ml H₂O gelöst, mit einer Mischung aus 1,2 ml 4 M Kaliumacetat und 24 ml Ethanol abs. gefällt, sedimentiert und schließlich erneut in 10 ml H₂O (1 ml pro g Gewebe) aufgenommen.

### Gewinnung von Poly(A)-haltiger Plazenta-mRNA

Die Plazenta-RNA wurde zur Gewinnung von Poly(A)-haltiger mRNA über Oligo(dT)-Cellulose-Chromatographie (Aviv and Leder, Proc. Natl. Acad. Sci. USA 69 (1973) 1408-1412) in 2 ml Pasteurpipetten in LiCl aufgetrennt. Etwa 5 mg Plazenta-RNA wurden in Puffer 1 (500 mM LiCl, 20 mM Tris (pH 7,5), 1 mM EDTA, 0,1 SDS) auf die Säule aufgetragen. Während die Poly(A)⁺-RNA an Oligo(dT)-Cellulose gebunden wurde, konnte die Poly(A)⁻-RNA wieder eluiert werden. Nach einem Waschschritt mit Puffer 2 (100 mM LiCl, 29 mM Tris (pH 7,5), 1 mM EDTA, 0,1 % SDS) wurde die Poly(A)⁺-RNA (Plazenta-mRNA) mit Puffer 3 (5 mM Tris (pH 7,5), 1 mM EDTA, 0,05 % SDS) von der Säule eluiert.

Zur weiteren Reinigung wurde die Poly(A)⁺-RNA auf Puffer 1 eingestellt und erneut über Oligo(dT)-Cellulose chromatographiert.
Die Ausbeute an Plazenta Poly(A)⁺-RNA betrug nach diesem zweiten Reinigungsschritt etwa 4 % der eingesetzten RNA.

### Synthese von cDNA aus humaner Plazenta (Plazenta-cDNA) und doppelsträngiger cDNA (dsDNA)

Poly(A)-haltige Plazenta-mRNA wurde vor der cDNA-Synthese im 1,5 % Agarosegel auf Intaktheit geprüft.

Danach wurden 4 µg Plazenta-mRNA in 65,5 µl H₂O gelöst, 10 min. bei 70°C denaturiert und im Eis wieder abgekühlt. Die cDNA-Synthese erfolgte in einem 100 µl-Ansatz nach Zugabe von 20 µl RT₁-Puffer (250 mM Tris (pH 8,2) bei 42°C, 250 mM KCl, 30 mM MgCl₂), 2,5 µl 20 mM dNTP (d.h. aller vier Desoxynukleosidtriphosphate), 1 µl Oligo(dT) von 1 µg/ml, 1 µl 1 M DTT, 2 µl RNAsin und 8 µl Reverse Transcriptase (24 U/µl) für 90 min. bei 42°C. Doppelsträngige cDNA (dsDNA) wurde nach Gubler and Hoffmann (Gene 25 (1983) 263-269) synthetisiert. Die Synthese erfolgte unmittelbar nach der cDNA-Synthese durch Zugabe von 305,5 µl H₂O, 80 µl RT 2 -Puffer (100 mM Tris (pH 7,5), 25 mM MgCl , 500 mM KCl, 50 mM DTT, 250 µg/ml BSA), 2 µl RNase H (2²U/µl), 2,5 µl E. coli DNA Ligase (5 U/µl), 5 µl 15 mM β-NAD, und 5 µl DNA Polymerase I (5 U/µl) und Inkubation für 5 h bei 15°C. Durch Hitzeinaktivierung (70°C, 30 min.) wurde die Reaktion beendet.

Der Reaktionsansatz wurde nach Zugabe von 55 µl 250 µM dNTP, 55 µl 10 mM Tris (pH 7,5), 10 mM MgCl₂, 10 µg/ml BSA, 3 µl T4 DNA-Polymerase I (1 U/µl), 2 µl RNase H (2 U/µl) und 2 µl RNase A (2 µg/ml) für weitere 30 min. bei 37°C inkubiert, um Fehlsynthesen der Polymerase I am zweiten DNA-Strang zu korrigieren ("Repair Reaction").

### Ligierung von EcoRI-Linkern an die dsDNA und Öffnen der Linker

Zur Errichtung einer Plazenta-cDNA-Bank wurde die dsDNA mit EcoRI-Enden versehen, um sie in die EcoRI-Schnittstelle des Phagenvektors λgt10 ligieren zu können (T. Maniatis et al (1982), Molecular Cloning, A Laboratory Manual, Cold Spring Harbor). Hierzu wurde die dsDNA
a) mit EcoRI-Methylase behandelt, um interne EcoRI-Schnittstellen der dsDNA zu schützen,
b) mit EcoRI-Linkern versehen, die
c) danach mit EcoRI geöffnet wurden.

### Zu a):

Die Methylase-Reaktion der dsDNA erfolgte direkt im Anschluß an die "Repair Reaction" nach Zugabe von 25 µl 500 mM EDTA (pH 8,0), 60 µl Methylase-Puffer (100 mM NaOAc (pH 5,2), 2 mg S-Adenosyl-L-Methionin) und 2 µl EcoRI-Methylase (20 U/µl) durch Inkubation bei 37°C für 30 min.
Der Reaktionsansatz wurde mit Phenol extrahiert und die dsDNA mit 60 µl 4M NaOAc und 1300 µl Ethanol gefällt. Die dsDNA wurde zweimal mit 70 % Ethanol gewaschen, einmal mit Ether ausgeschüttelt und getrocknet.

### Zu b):

Die EcoRI-methylierte dsDNA wurde in 88 µl H₂O gelöst und nach Zugabe von 10 µl Ligase-Puffer (500 mM Tris (pH 7,4), 100 mM MgCl₂, 100 mM DTT, 10 mM Spermidin, 10 mM ATP, 1 mg/ml BSA), 1 µl T4 DNA-Ligase (10 U/µl) mit 1 µl EcoRI-Linkern (0,5 µg/µl) (pGGAATTCC bzw. pAGAATTCT) über Nacht bei 15°C ligiert.

### Zu c):

Das Volumen des Ligase-Ansatzes wurde mit 6 µl H₂O, 12 µl 10x EcoRI-Puffer und 2 µl EcoRI (120 U/µl) auf 120 µl gebracht. Die EcoRI-Verdauung erfolgte für 2 h bei 37°C.

### Abtrennen nichtgebundener Linker über Kaliumacetat-Gradienten und Größenselektionierung der dsDNA

Zur Abtrennung aller nichtgebundenen EcoRI-Linker von der dsDNA wurde der EcoRI-Reaktionsansatz in toto auf einen Kaliumacetat-Gradienten (5-20 % KOAc, 1 mM EDTA, 1 µl/ml Ethidiumbromid) aufgetragen und 3 h bei 50 000 rpm und 20°C zentrifugiert (Beckman SW 65-Rotor).
Der Gradient wurde von unten so fraktioniert, daß die ersten fünf Fraktionen 500 µl maßen und alle restlichen 100 µl. Die Fraktionen wurden mit 0,01 Volumen Acrylamid ( 2 mg/ml) und 2,5 Volumen Ethanol gefällt, einmal mit 70 %igem Ethanol gewaschen, getrocknet und jeweils in 5 µl H₂O aufgenommen.

Zur Größenbestimmung der dsDNA wurden 1 µl jeder Fraktion im 1,5 % Agarose-Gel analysiert. Zusätzlich wurde mit 1 µl jeder Fraktion die Quantität an dsDNA bestimmt.

Fraktionen mit dsDNA über 1000 bp wurden vereinigt und die Probe so eingeengt, daß die Endkonzentration 27 µg/ml betrug.

### Einbau der dsDNA in den Phagenvektor λgt10 und "in vitro packaging"-Reaktion

Der Einbau der dsDNA in die EcoRI-Schnittstelle des Phagenvektors λgt10 (Vector Cloning Systems, San Diego, CA) erfolgte in einem Ligaseansatz von 4 µl: 2 µl dsDNA, 1 µl λgt10 x EcoRI (1 µg/ml), 0,4 µl Ligase-Puffer, 0,5 µl H₂O, 0,1 µl T4 DNA-Ligase. Der Ansatz wurde 4 h bei 15°C inkubiert.

Zur Etablierung der Plazenta-cDNA-Bank im Phagenvektor λgt10 folgte mit den λ-lysogenen Zellextrakten E. coli NS 428 und NS 433 eine "in vitro packaging"-Reaktion des Ligaseansatzes bei Raumtemperatur für 2 h (Vector Cloning Systems, San Diego, CA; Enquist and Sternberg, Methods in Enzymology 68, (1979), 281-298). Die Reaktion wurde mit 500 µl Suspensionsmedium (SM: 0,1 M NaCl, 8 mM MgSO₄, 50 mM Tris (pH 7,5), 0,01 % Gelatine) und 2 Tropfen Chloroform gestoppt.

### Titerbestimmung und Analyse der Plazenta-cDNA-Bank

Die Zahl der "Plaque Forming Units" (PFU) der Plazenta-cDNA-Bank wurde mit kompetenten Zellen des E. coli- K 12 - Stammes C600 HFL bestimmt: Sie betrug 1 x 10⁶ PFU. Etwa 80 % aller Phagen enthielten DNA-Inserts, die größer als 1000 Basenpaare waren.

### Oligonukleotidsonden zum "Screening" der Plazenta-cDNA-Bank

Zwei Oligonukleotidsonden (20mer-Sonde und 66mer-Sonde) wurden zur Analyse der Plazenta-cDNA-Bank synthetisiert. Ihre Sequenzen wurden abgeleitet von der Aminosäureprimärsequenz mehrerer proteolytischer bzw. BrCN-Fragmente von F XIIIa. In einigen Fällen wurden überlappende, dadurch längere Aminosäuresequenzen ermittelt, die die Synthese einer sehr langen Probe, nämlich der 66mer-Sonde, erlaubte.

Die 20mer-Sonde ist eine konventionelle DNA-Sonde, bei der alle theoretisch möglichen Codons für die Aminosäuresequenz Met-Met-Asp-Ile-Thr-Asp-Thr berücksichtigt wurden (im Falle der letzten Aminosäure, Thr, wurde die 3. Position im Codon, die sog. "Wobble"-Position, weggelassen; siehe Tabelle 1). Die 20mer-Sonde ist somit 48fach degeneriert, d.h. ein Gemisch aller 48 theoretisch möglichen codierenden Oligonukleotide der genannten Aminosäuresequenz.

Art des Aufbaues und der Verwendung der 66mer-Sonde folgte im wesentlichen den Regeln von Lathe, (a. a. O.). Um die 66mer-Sonde aufzubauen, wurden zwei 39mer-Sonden synthetisiert (39mer A mit der Sequenz: 5' TATGGCCAGTTTGAGGATGGCATCCTGGACACCTGTCTG 3' und 39mer B mit der Sequenz:
5' GTCCATCTGGGCCCGGTCCATCACATACAGACAGGTGTC 3'). 39mer A und 39mer B weisen eine 12 Basen umfassende komplementäre Sequenz auf, so daß nach Hybridisierung der beiden Sequenzen lange, freie 5'-Enden entstehen.

Die beiden 39mer-Sonden wurden (ebenso wie die 20mer Sonde) mit T4 Polynukleotidkinase unter Anwesenheit von (γ-³²P) ATP am 5'-Ende markiert (ca. 1 µg DNA, (γ-³²P) ATP: 3000 Ci/mmol, 10 µCi/µl, eingesetzt wurden 6 µl/40 µl Reaktionsansatz). Die 20mer-Sonde hatte eine spez. Aktivität von 1 x 10⁸ Bq/µg bzw. 1,5 x 10 Bq/pMol. Die beiden 39mer-Sonden wurden für 5 min. auf 95°C erhitzt, zusammengegeben, langsam im Kühlraum auf 4°C abgekühlt und dadurch hybridisiert. Anschließend wurde ca 1 µg der hybridisierten 39mer-Sonden mit 32 DNA-Polymerase I, Klenow-Fragment, unter Zusatz von (α-³²P) dATP (3000 Ci/mmol, 10 µCi/µl, 4 µl/50 µl Reaktionsansatz) behandelt (5' → 3'-Auffüllreaktion). Die aufgefüllte 66mer-Sonde hatte eine spez. Aktivität von 1,5 x 10⁸ Bq/µg. Die DNA-Sonden wurden bei -20°C gelagert; die 20mer-Sonde wurde zur Analyse (Screening) direkt eingesetzt, die 66mer-Sonde wurde zuvor 5 Min. auf 95°C erhitzt und anschließend schnell im Eisbad abgekühlt.

Da die 66mer-Sonde durch Hybridisierung zweier "39mere" mit anschließender 5' → 3'-Auffüllreaktion zustande kam, lassen sich verschiedene Versuche durchführen. Zum einen können cDNA-Banken mit der denaturierten 66mer-DNA-Sonde hybridisiert werden, zum anderen können positive Klone anschließend mit den 39mer-A- und B-Sonden einzeln hybridisiert werden.

Solche Klone, die sowohl mit der langen als auch mit beiden kurzen 39mer-Sonden A und B hybridisieren, besitzen mit großer Wahrscheinlichkeit die gesuchte Sequenz. Die beschriebene Methode des Aufbaues einer langen, komplexen Oligonukleotidprobe und des "Nachscreenens" der Klone mit Hilfe der partiell komplementären, kurzen DNA-Sonden stellt somit eine Erhöhung der Spezifität dar. Zudem können mit dem langen Oligonukleotid bzw. dessen partiell komplementären, kurzen Teil-Oligonukleotiden genomische Banken mit erhöhter Spezifität "gescreent" werden. Ein weiterer Vorteil der genannten Methode besteht darin, daß die Synthese kürzerer Oligonukleotide leichter und mit höherer Ausbeute und Genauigkeit durchführbar ist. Durch die Folge von zwei enzymatischen Reaktionen, nämlich 1) T4 Polynukleotid-Kinase zur (γ-³²P) ATP-Markierung und 2) DNA-Polymerase-Auffüllreaktion unter (α - ³²P) dNTP Zugabe, lassen sich höhere spezifische Aktivitäten (≥ 1x10⁸ Bq/µg DNA) erzielen.

### "Screening" der Plazenta-cDNA mit F XIIIa-spezifischen Oligonukleotiden

5 x 10⁵ PFU der Plazenta-cDNA-Bank wurden mit der 20mer-Sonde und der 66mer-Sonde nach F XIIIa-codierenden cDNA-Sequenzen untersucht. Dazu wurden 3 x 10⁴ PFU mit Zellen des E. coli K 12-Stammes C 600 HFL in Weich-Agar auf 13,5 cm-Petrischalen ausplattiert und 6 h bei 37°C inkubiert. Zu diesem Zeitpunkt war noch keine vollständige Lyse eingetreten. Die Platten wurden über Nacht im Kühlschrank inkubiert und die Phagen auf Nitrocellulosefilter (Schleicher & Schüll, BA 85, Ref.-Nr. 401124) übertragen (Duplikate). Nitrocellulosefilter und Petrischalen wurden mit einer Injektionskanüle markiert, um eine spätere Zuordnung zu ermöglichen. Die Petrischalen wurden während des Prozessierens der Nitrocellulosefilter im Kühlraum gelagert. Die auf den Nitrocellulosefiltern befindliche DNA wurde denaturiert, indem die Filter für 5 min. auf mit 1,5 M NaCl, 0,5 M NaOH-getränktes Filterpapier (Whatman-M 3) gelegt wurden. Anschließend wurden die Filter auf die gleiche Art mit 1,5 M NaCl, 0,5 M Tris (pH 8,0) renaturiert und mit 2 x SSPE (0,36 M NaCl, 16 mM NaOH, 20 mM NaH₂ PO₄, 2 mM EDTA) gewaschen. Die Filter wurden dann für 2 h bei 80°C im Vakuum getrocknet. Die Filter wurden für 4 h bei 65°C in 3xSSC, 0,1 % SDS (20 x SSC = 3 M NaCl, 0,3 M Na-Citrat) gewaschen und für 4 h bei 65°C vorhybridisiert (Prähybridisierungslösung: 0,6 M NaCl, 0,06 M Tris (pH 8,3), 6 mM EDTA, 0,2 % nichtionisches synthetisches Saccharose-Polymer, ( Ficoll), 0,2 % Polyvinylpyrrolidon 40, 0,2 % BSA, 0,1 % SDS, 50 µg/ml denaturierte Heringssperma-DNA). Die Filter wurden über Nacht unter Zusatz von 100 000-200 000 Bq des markierten Oligonukleotids/ml Hybridisierungslösung (wie Prähybridisierungslösung, jedoch ohne Heringssperma-DNA) in Bechergläsern bzw. in zugeschweißten Polyethylenfolien unter leichtem Schütteln inkubiert. Die Hybridisierungstemperatur für die 20mer-Sonde und für die 39mer-Sonden betrug 42°C und für die 66mer-Sonde 47°C. Die Nitrocellulosefilter wurden mit 6 x SSC, 0,05 M Natriumpyrophosphat eine Stunde bei Raumtemperatur und für eine weitere Stunde bei der jeweiligen Hybridisierungstemperatur gewaschen. Die Filter wurden getrocknet und über Nacht autoradiographiert. Signale auf dem Röntgenfilm, die auf beiden Duplikaten auftraten, wurden der Petrischale zugeordnet und die Region (ca. 50 Plaques) mit dem breiten Ende einer Pasteurpipette ausgestanzt und die Phagen in 1 ml SM-Puffer resuspendiert. Positive Phagen wurden über drei Runden vereinzelt, bis ein einzelner Klon vorlag.

Insgesamt wurden 5 x 10⁵ PFU der Plazenta-cDNA-Bank in mehreren Durchgängen untersucht. Es wurden 17 Signale auf Duplikat-Filtern identifiziert. Beim weiteren Screening unter stringenteren Bedingungen blieben 7 Signale positiv. Von diesen 7 PFU zeigte ein einziger PFU ein positives Signal sowohl nach Hybridisierung mit der 20mer- und 66mer-Sonde als auch mit den 39mer-Sonden A und B. Dieser Klon - im folgenden λgt10-12 genannt - trägt eine für F XIIIa codierende Sequenz von 1704 Basenpaaren mit einer internen EcoRI-Schnittstelle. Das kleinere EcoRI-Fragment von 540 Basenpaaren hybridisiert im "Southern Blot" mit der 20mer-DNA-Sonde und das größere Fragment von 1164 Basenpaaren mit der 66mer-DNA-Sonde.

Beim Nachscreening stellte sich im "Southern Blot" heraus, daß die Reaktion mit der 39mer-Sonde A stärker ist als mit der 39mer-Sonde B. Die Sequenzanalyse des Klones λgt10-12 zeigte im folgenden, daß über die gesamte Länge der 66mer-Sonde lediglich sieben "Mismatches" zur ermittelten F XIIIa-Sequenz auftreten (Tabelle 2). Die sieben "Mismatches" verteilen sich wie folgt: In der 39mer-A-Sonde treten drei, in der 39mer-B-Sonde treten fünf "Mismatches" auf (ein "Mismatch" tritt in der überlappenden Region, also bei beiden gemeinsam auf). Die fünf "Mismatches" der 39mer-B-Sonde treten "geclustert" auf, was möglicherweise der Grund für die schwächeren Hybridisierungssignale im Falle der 39mer-B-Sonde ist.

### Screening der Plazenta-cDNA mit "nick-translated" EcoRI-Fragmenten

Die beiden subklonierten 540 Basenpaare und 1164 Basenpaare langen EcoRI-Fragmente wurden in die EcoRI-Schnittstelle des handelsüblichen Vektors pUC8 kloniert (1164 bp ≙ pUC8-12.1 und 540 bp ≙ pUC8-12.2) und daraus mit EcoRI präparativ isoliert und in Anwesenheit von (α - ³²P) dNTP "nick-translated". Die spezifische Aktivität der beiden Fragmente betrug 1x10⁸ Bq/µg DNA. Mit Hilfe der (³²P)-markierten Fragmente wurden in mehreren Durchgängen ca. 1 x 10⁶ rekombinante Phagen der Plazenta-cDNA-Bank untersucht (Hybridisierungstemperatur 65°C) und dabei 13 hybridisierende Phagen identifiziert. Die Phagen wurden über drei Runden vereinzelt, bis eine homogene, singuläre Phagenpräparation vorlag. Von jedem Phagen wurden 20 ml Lysate angelegt und DNA extrahiert. Die DNA wurde mit EcoRI verdaut und auf einem 1 % Agarosegel aufgetrennt. Das Gel wurde der "Southern-Blot"-Technik unterzogen und der Nitrocellulosefilter mit dem markierten 540 bp EcoRI-Fragment hybridisiert. Elf Phagen zeigten Hybridisierungssignale. Der Nitrocellulosefilter wurde gekocht und mit dem "nick-translated" 1164 bp EcoRI-Fragment hybridisiert. Neun Phagen zeigten Hybridisierungssignale.

Aufgrund der Größe der hybridisierenden Fragmente konnten Klone identifiziert werden, die sowohl zum 5'-Ende wie λgt10-20 als auch zum 3'-Ende wie λgt10-11 im Vergleich zu λgt10-12 expandieren. Mit Hilfe dieser Klone konnte die vollständige F XIIIa-cDNA-Sequenz ermittelt werden. Vorhandene Teilsequenzen konnten unter Benutzung interner Restriktionsschnittstellen oder durch Hybridisierung überlappender Sequenzen zusammengesetzt werden. Die vollständige cDNA-Sequenz kann in Expressionsvektoren einligiert werden und in geeigneten pro- oder eukaryontischen Systemen exprimiert werden.

### DNA-Sequenzanalyse

Der Phagenklon λgt10-12 wurde vermehrt und DNA extrahiert. Die beiden EcoRI-Fragmente wurden isoliert und in die EcoRI-Stelle des Plasmid-Vectors pUC8 kloniert. pUC8-12.1 trägt.das 1164 Basenpaare Fragment, pUC8-12.2 trägt das 540 Basenpaare Fragment. Um das gesamte, 1704 Basenpaare umfassende Fragment zu isolieren, wurde λgt10-12 partiell mit EcoRI verdaut, die 1704 Basenpaare-Bande isoliert und in die EcoRI-Stelle von pIC19 H (Marsh et al., Gene 32 (1984) 481-485) kloniert. Das resultierende Plasmid heißt pIC19H-12.

Durch Klonierung von Sau 3A-, AluI- und TaqI-Subfragmenten der Klone pUC8-12.1, pUC8-12.2 und pIC19H-12 in pUC-Plasmide und M13-Phagen und nachfolgender Sequenzierung der relevanten Regionen mit der enzymatischen Didesoxymethode nach Sanger und der chemischen Methode nach Maxam und Gilbert konnte die Sequenz des 1704 bp-Fragmentes ermittelt werden (Tabelle 3). Die Sequenz zeigt nur einen offenen Leserahmen und codiert für die ersten 542 Aminosäuren des Faktor XIIIa-Moleküls.

Die Restriktionsanalyse von Klon λgt10-12 bzw. pIC19H-12 sowie der Klone λgt10-11 und λgt10-20 wurde sowohl durch geeignete Einzel- und Mehrfachverdauungen als auch durch partielle Verdauung (³²P)-markierter DNA-Fragmente nach der Methode von Smith und Birnstiel durchgeführt (Smith, H.O. and Birnstiel, M.L., Nucleic Acids Res. 3 (1976) 2387-2398) (Figur 1).

Der Klon λgt10-11 trägt ein 2432 bp großes Fragment mit einer internen EcoRI-Schnittstelle. Dieses Fragment überlappt am 3'-Ende das cDNA-Fragment aus λgt10-12 um 237 bp und umfaßt die restlichen 570 bp der codierenden Sequenz sowie 1625 bp der nicht codierenden Region einschließlich einer Poly(A)-Sequenz von 89 Basen. Der Klon λgt10-20 mit einem etwa 700 bp großen cDNA-Fragment trägt am 5'-Ende zusätzlich noch 6 Basen mehr (GAG GAA...) als λgt10-12.

### 2. Herstellen eines exprimierbaren, die vollständige codierende F XIIIa-cDNA enthaltenden Klones

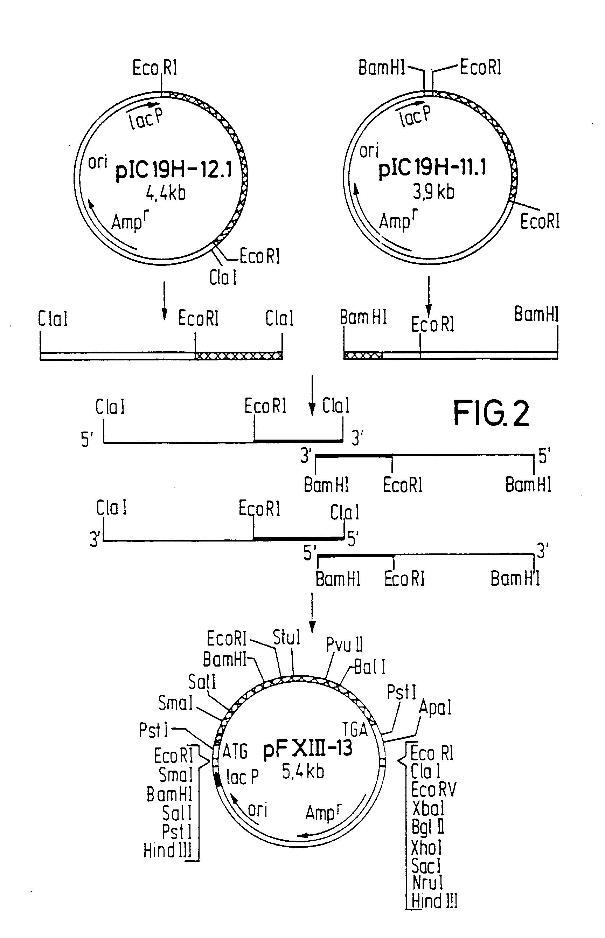

Um zu einem Plasmid zu gelangen, das den vollständigen codierenden Abschnitt der F XIIIa-cDNA enthält, wurden die Ausgangsklone λgt10-11 und λgt10-12 herangezogen. Mit Hilfe von partieller EcoRI-Verdauung wurde die 1704 Basenpaare umfassende Insertion von λgt10-12 in die EcoRI-Stelle von pIC19H (Marsh et al., a. a. O.) kloniert. Im weiteren wurde das resultierende Plasmid pIC19H-12.1 verwendet (siehe Fig. 2). Der Klon λgt10-11 trägt eine 2432 Basenpaare große Insertion mit einer internen EcoRI-Schnittstelle. Das linke ("5'-endige") 1224 Basenpaare große EcoRI-Fragment, das die C-terminalen 570 Basenpaare der codierenden Sequenz sowie 654 Basenpaare der 3'-nichtcodierenden Region umfaßt, wurde ebenfalls in die EcoRI-Schnittstelle von pIC19H kloniert. Im weiteren wurde das resultierende Plasmid pIC19H-11.1 verwendet (Fig. 2). Die Plasmide pIC19H-12.1 und pIC19H-11.1 tragen die codierende Region für F XIIIa in der gleichen Orientierung im Vektor und weisen eine 237 Basenpaare umfassende überlappende Region auf. Diese überlappende Region wurde dazu benutzt, um aus den Teilklonen pIC19H-12.1 und pIC19H-11.1 einen Klon zu erstellen, der die vollständige codierende Region umfaßt. Dazu wurde aus den beiden genannten Plasmiden durch Hybridisierung in vitro partiell einzelsträngige Heteroduplexmoleküle hergestellt (Fig. 2) und die Reaktionsmischung in E. coli transformiert. Durch Ausnutzung der Reparaturmechanismen des Bakteriums (3'→ 5'-Exonukleaseaktivität, 5'→ 3'-Polymerisationsaktivität des Enzyms DNA-Polymerase I) wurde ein Plasmid mit vollständiger codierender Region erhalten.

Es wurden dazu im einzelnen jeweils 1 µg DNA der Plasmide pIC19H-12.1 (ClaI-verdaut) und pIC19H-11.1 (BamHI-verdaut) gemischt und mit Ethanol gefällt. Die DNA wurde in vacuo getrocknet, in 20 µl H₂O aufgenommen und nach Zugabe von 5 µl 1 N NaOH 10 Minuten bei Raumtemperatur inkubiert. Anschließend wurden in folgender Reihenfolge zugesetzt: 200 µl H₂O, 25 µl 1 M Tris-HCl (pH 8,0), und 50 µl 0,1 N HCl. Der Reaktionsansatz wurde für 3 Stunden bei 65°C inkubiert, mit Ethanol gefällt und getrocknet. Die DNA wurde in 20 µl H₂O resuspendiert und nach bekannten Methoden in E. coli transformiert, die Zellen auf LB-Amp-Platten plattiert und über Nacht inkubiert. Von den insgesamt 96 ampicillinresistenten Klonen wurden 24 Klone nach der Alkali-Methode (Birnboim und Doly, Nucl. Acid Res. 7 (1979) 1513-1523) aufgearbeitet und die Plasmide mit HindIII, EcoRI, BamHI und PvuII restriktionsendonukleolytisch charakterisiert. Sechs Plasmide zeigten das zu erwartende Restriktionsmuster. Eines dieser Plasmide, pFXIII-13 (Fig. 2), wurde näher charakterisiert. Dazu wurde das StuI (Position 1225)-PvuII (Position 1870)-Fragment, das die 237 Basenpaare überlappende Region umfaßt, sequenziert und die korrekte DNA-Sequenz bestätigt. Das Plasmid pFXIII-13 umfaßt 2693 Basenpaare FXIIIa-cDNA, davon 78 bp der 5'-nichttranslatierten Region, 2196 bp vollständige Codierungsregion sowie 419 bp der 3'-nichttranslatierten Region. pFXIII-13 war das Ausgangsplasmid für alle folgenden Expressionsversuche.

### 3. Expression von biologisch aktivem Faktor XIIIa in E. coli

### a) Konstruktion von F XIIIa-Expressionsplasmiden

pFXIII-13 trägt das F XIIIa-cDNA-Insert in der korrekten Orientierung zum lac-Promoter und im korrekten Leserahmen zum lacZ α-Peptid. pFXIII-13 ist somit in der Lage, die Synthese eines F XIIIa-Fusionsproteins in E. coli zu induzieren. Das Molekulargewicht dieses Proteins umfaßt die 732 Aminosäuren des natürlichen F XIIIa, zusätzlich 16 vektorcodierte Aminosäuren sowie 28 Aminosäuren, die durch die 5'-nichtcodierende Region spezifiziert werden. Diese zusätzlichen 44 Aminosäuren befinden sich am N-terminalen Ende des Fusionsproteins. Das erwartete Molekulargewicht dieses Proteins beträgt 85 250 D (Tab. 4).

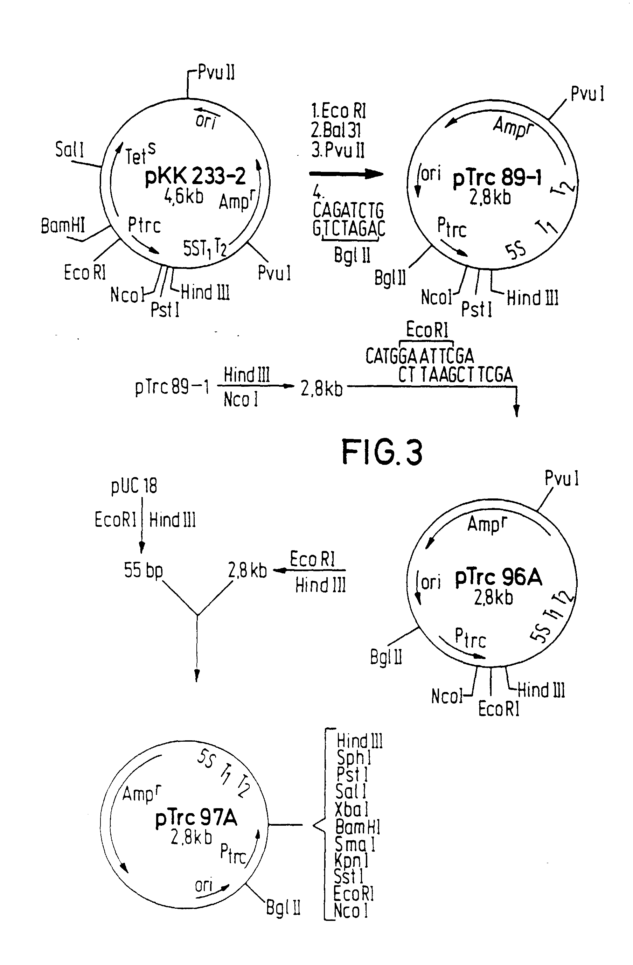

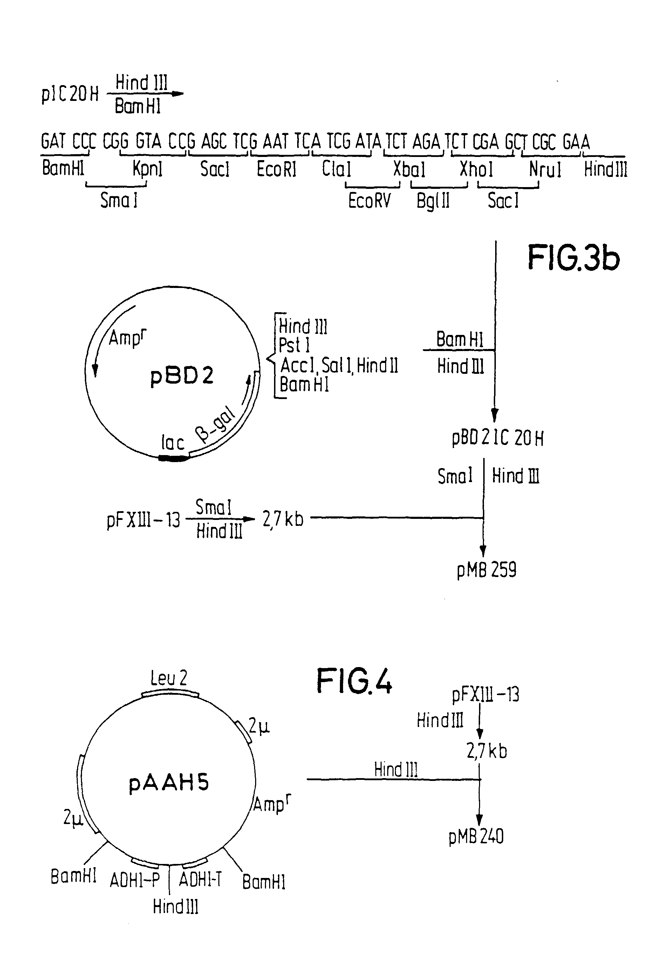

Im weiteren wurde ein Expressionsplasmid konstruiert, welches anstelle des lac-Promotors einen effizienteren trp/lac-Hybridpromotor benutzt. pKK233-2 (Amann und Brosius, Gene 40 (1985), 183-190) wurde dazu mit EcoRI geschnitten und mit Bal31 behandelt (Fig. 3). Anschließend wurde die DNA mit PvuII geschnitten und das 2800 Basenpaare große Fragment über ein PAA-Gel gereinigt. Dieses Fragment wurde in Anwesenheit eines BglII-Linker (5' CAGATCTG) religiert. Das resultierende Plasmid pTrc89-1 (Fig. 3) wurde mit NcoI und HindIII geschnitten und der synthetische Linker 
5' CATGGAATTCGA 3'
3' CTTAAGCTTCGA 5'
 zusammen mit dem 2800 Basenpaar-Fragment in einem Ligaseansatz inkubiert. Das resultierende Plasmid, pTrc96A (Fig. 3), wurde mit EcoRI und HindIII geschnitten, das 2800 Basenpaare große Fragment Gel-gereinigt und mit dem 55 Basenpaare großen EcoRI-HindIII-Linker aus pUC18 ligiert. Das resultierende Plasmid ist pTrc97A (Fig. 3). pTrc97A trägt - im Gegensatz zu pKK233-2 - hinter der im Plasmid nur einmal auftretenden NcoI-Stelle den Polylinker aus pUC18 und somit zahlreiche Klonierungsstellen.

Die in pFXIII-13 klonierte FXIIIa-cDNA trägt 21 Basenpaare 5' vom ATG-Initiationscodon entfernt eine PstI-Stelle (Position 61). Die nächste PstI-Stelle befindet sich in der 3'-untranslatierten Region (Position 2398). Das 2337 Basenpaare lange PstI-Fragment wurde aus pFXIII-13 isoliert und in die PstI-Stelle von pTrc97A ligiert. Das resultierende Plasmid mit dem PstI-Fragment in der gewünschten Orientierung ist pFXIII-C4 (Fig. 3a). Das erwartete durch pFXIII-C4 spezifizierte FXIII-Molekül trägt 22 zusätzliche N-terminale Aminosäuren, wovon 15 vectorcodiert und 7 durch die 5'-nichtcodierende Region der F XIII-cDNA spezifiziert werden. Das erwartete Molekulargewicht dieses Proteins beträgt 82 830 D (Tab. 4).

Durch Abspalten der aminoterminalen 37 Aminosäuren durch Thrombin wird der F XIIIa in die aktive Form umgesetzt. Da solch ein bereits aktiviertes F XIIIa-Molekül therapeutisch interessant ist, wurde versucht, einen durch Thrombinspaltung verkürzbaren F XIIIa in E. coli zu exprimieren. Um eine hohe Ausbeute zu erreichen, wurde die Klonierung so vorgenommen, daß der verkürzte F XIIIa in Form eines Hybridproteins, fusioniert an ein E. coli-β-Galaktosidasefragment, exprimiert wird. Das ca. 2700 bp große SmaI-HindIII-Fragment aus pFXIII-13 wurde isoliert und in pBD2IC20H ligiert, das mit SmaI und HindIII hydrolysiert worden war. In dem neuen Plasmid pMB259 (Fig. 3b) befindet sich die codierende Region der F XIIIa-cDNA von der Aminosäure Pro₃₇ bis Met₇₃₂ im Leserahmen zu den aminoterminalen 375 Aminosäuren der β-Galactosidase und verfügt über die Thrombin-Spaltstelle Arg₃₈/Gly₃₉, so daß aus den synthetisierten Fusionsproteinen aktivierter F XIIIa durch Thrombin-Spaltung erhalten werden kann.

Der Expressionsvektor pBD2IC20H war konstruiert worden, indem die 58bp umfassende Polylinkerregion des Plasmides pIC20H (Marsh et al., a. a. O.) als BamHI-HindIII-Fragment in pBD2 (Bröker, Gene Anal. Techn. 3 (1986) 53-57), das den lac-Promotor trägt, ligiert worden war.

### b) Expression

Es wurde gefunden, daß E. coli-Zellen des Stammes D29A1, die mit pFXIII-13, mit pFXIII-C4 oder mit pMB259 transformiert werden, in der Lage sind, die erwarteten F XIIIa-Proteine zu synthetisieren. Die Expression von F XIII von den Plasmiden pFXIII-13, pMB 259 und pFXIII-C4 kann mit Hilfe von IPTG induziert werden. Der Vergleich von Protein-Extrakten nach IPTG-Induktion von E. coli-D29A1 (pFXIII-13) und D29A1 (pFXIII-C4) auf mit Coomassie-Blau gefärbten PAA-Gelen ergab die erwarteten Molekulargewichte der F XIII-Fusionsproteine. Die geschätzte Expression des "44aa-FXIII-Fusionsproteins" ist ca. 5 mal höher als die des "22aa-FXIII-Fusionsproteins".

Weiterhin wurde gefunden, daß die durch pFXIII-13 und pFXIII-C4 spezifizierten F XIIIa-Moleküle biologische Aktivität besitzen. In E. coli D29A1-Kontrollextrakten wurde dagegen keine FXIII-Aktivität gefunden. Die im 'Clot stability assay' (Karges, in Bergmeier, Methods of Enzymatic Analysis, Volume 5, Enzymes 3: Peptidases, Proteinases and their Inhibitors, Seite 400-410) gefundene Aktivität für E. coli D29A1 (pFXIII-13) ist 5 µg/l, bezogen auf die E. coli-Kultur (OD₂₅₀=1,5), und für D29A1 (pFXIII-C4) 15 µg/l.

Die im F XIIIa-spezifischen ELISA gefundene Menge an Faktor XIII beträgt 1,5 mg/l, bezogen auf die E. coli-Kultur, für pFXIII-13 und 3 mg/l für pFXIII-C4. Die Diskrepanz zwischen der im biologischen Test und der im ELISA gemessenen Menge an F XIII beruht darauf, daß der größte Teil (> 90 %) an F XIII als unlösliches Präzipitat in der E. coli-Zelle auftritt, welches nicht biologisch aktiv ist und erst in 7 M Harnstoff in Lösung geht. Die lösliche Fraktion der in E. coli-Extrakten vorhandenen F XIII-Moleküle zeigt im Ouchterlony-Test (Ouchterlony, Progr. Allergy 5 (1958) 1) eine weitgehend identische Präzipitationskurve im Vergleich zu aus Plazenta isoliertem F XIII.
Die Expression von eukaryontischen Proteinen in E. coli in Form von unlöslichen Protein-Aggregaten wurde bereits für mehrere Proteine beschrieben. Diese Proteine können aus solchen Aggregaten mit Hilfe eines Chaotrops herausgelöst werden und durch geeignete Renaturierungsbedingungen in ihre biologisch aktive Form überführt werden.

### 4. Expression von F XIII in Hefen

Die Synthese von gentechnisch gewonnenem biologisch aktivem F XIII aus Hefen wurde dadurch erreicht, daß die für F XIII codierende cDNA in Expressionsvektoren eingebaut wurde, die sich in Hefen autonom replizieren können. Aus Extrakten der rekombinanten Klone konnte F XIII-aktives Protein isoliert werden.

Die Wachstumsbedingungen für Hefen sowie die molekularbiologischen Methoden sind beschrieben in Dillon et al., Recombinant DNA Methodology, John Wiley & Sons, New York (1985) sowie in Maniatis et al., a. a. O.

Die F XIII-cDNA wurde als ca. 2700 bp großes HindIII-Fragment aus dem Vektor pFXIII-13 isoliert und in die HindIII-Stelle des Vektors pAAH5 (Ammerer, Meth. Enzymol. 101 (1983), 192-201) kloniert. In dem erhaltenen Plasmid pMB240 (Fig. 4) steht die F XIII-cDNA somit unter der Kontrolle des starken ADHI-Promotors, der die Genexpressionssignale der Alkoholdehydrogenase enthält. Das Plasmid pMB240 wurde in die Bäckerhefe Saccharomyces cerevisiae, Stamm Leu 2-3, Pep 4-3, nach der Methode von Itoh et al. (J. Bacteriol. 153 (1983), 163-168) transformiert und Leu⁺-Transformanten auf YNB-Minimalmedium selektioniert. Eine Kolonie von transformierten Hefezellen wurde benutzt, um eine Flüssigkultur mit YNB-Medium anzuimpfen. Nach zweitägigem Wachstum bei 30°C wurde auf komplexes YPB-Medium überimpft, die Zellen nach weiteren drei Tagen abzentrifugiert und mit einer Glaskugelmühle in isotonischer Kochsalzlösung mit 100 mM Natriumcitrat (pH 7,2) aufgeschlossen. Der Zellextrakt wurde hochtourig 1 Stunde bei 20 000 UpM in der Sorvall-Hochgeschwindigkeitszentrifuge im SS34-Rotor bei 4°C zentrifugiert.

Der zellfreie Überstand von S. cerevisiae (pMB240) wurde im "Western blot"-Verfahren analysiert. Zusätzlich zu einer Bande, die in der gleichen Position wie F XIII aus Plazenta detektiert werden kann, reagierte ein Protein von ca. 116 000 D spezifisch mit dem eingesetzten anti-F XIII-Serum. Somit ist erwiesen, daß ein Teil des in Hefen gebildeten F XIII glykosyliert wird.
Die Glykosylierung von Proteinen ist häufig ein Faktor, der die Halbwertszeit von Proteinen, insbesondere von Plasmaproteinen, verlängert. Zudem können Kohlenhydrat-Seitenketten die Aktivität von Plasmaproteinen, z. B. des Antithrombin III, verstärken bzw. die Wirksamkeit verlängern.

Ein in Hefen exprimierter F XIII, der im Gegensatz zu aus Plazenta gewonnenem F XIII glykosyliert oder auf andere Art und Weise posttranslational modifiziert ist, kann durch erhöhte Aktivität Vorteile gegenüber F XIII aus Plazenta besitzen.

Der zellfreie Überstand wurde mit einem ELISA auf F XIII überprüft und, bezogen auf die Hefekultur, eine F XIII-Konzentration von 150 ng/ml festgestellt. Die biologische Aktivität von F XIII wurde nach Karges, a. a. O., ermittelt und bestätigte die im ELISA gemessenen Konzentrationen. Eine unspezifische F XIII-ähnliche Aktivität durch Hefeproteine konnte ausgeschlossen werden, da die biologische Aktivität des aus Bäckerhefe gewonnenen F XIII durch anti-F XIII-Antikörper spezifisch hemmbar war.

### 5. Expression von F XIIIa in animalen Zellen

### a) Konstruktion von Expressionsvektoren für animale Zellen

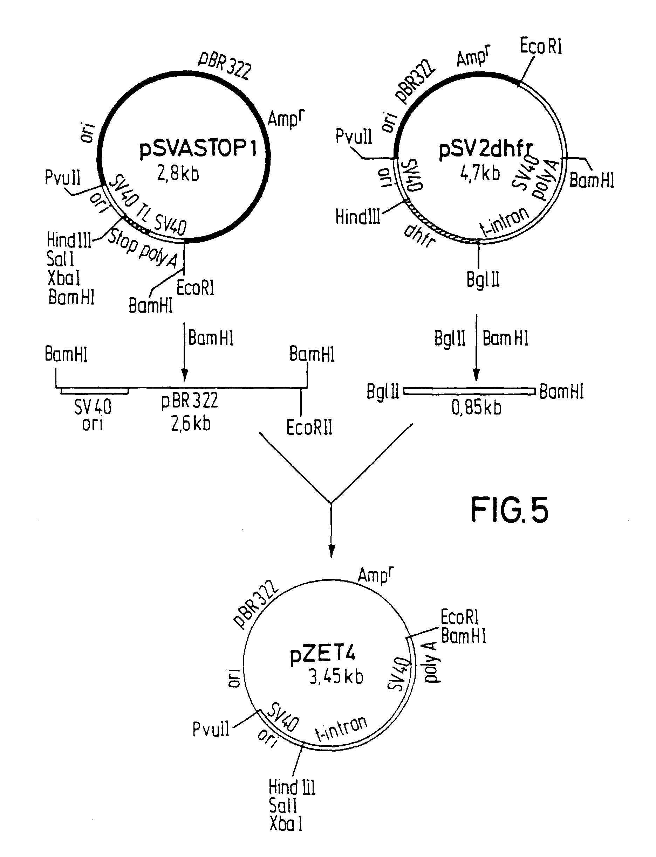

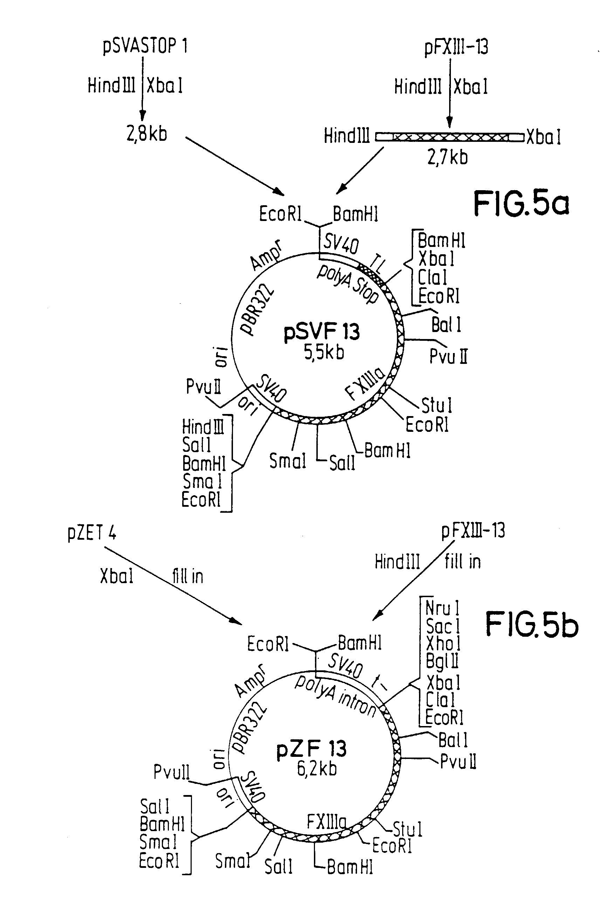

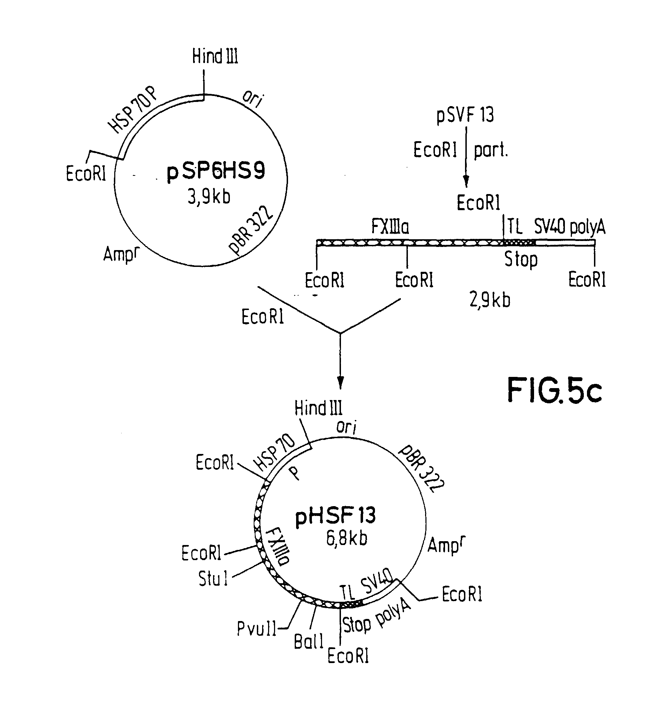

Der Expressionsvektor pSVA STOP1 ist in der deutschen Patentanmeldung P 36 24 453.8 vorgeschlagen (das Beispiel 1 dieser Anmeldung, das die Synthese dieses Vektors betrifft, ist auszugsweise im Anhang wiedergegeben). Zur Expression von F XIIIa in animalen Zellen wurden neben diesem Plasmid auch die Vektoren pZET4 (s. u.) und pSP6HS9, welches den Drosophila "heat shock protein 70"-Promotor trägt (Wurm et al., Proc. Natl. Acad. Sci. USA 83 (1986) 5414-5418) verwendet.
pZET4 (Fig. 5): Das Plasmid pSVA STOP1 wurde mit BamHI geschnitten und das 2,6 kb große Vektorfragment, das den SV40 "early"-Promotor trägt, wurde isoliert. In den derart vorbehandelten Vektor wurde das 0,85 kb große BglII-BamHI-Fragment aus pSV2dhfr (Lee et al., Nature 294 (1981) 228-232) ligiert, was zu dem Plasmid pZET4 führt. Auf dem 0,85 kb-Fragment aus pSV2dhfr befinden sich mRNA-"splice sites" aus Exon-Intron-Übergängen und die Polyadenylierungsstelle des Gens für das t-Antigen aus der SV40-DNA.

### b) Konstruktion von F XIIIa-Expressionsvektoren für animale Zellen

pSVF13 (Fig. 5a): Der Expressionsvektor pSVA STOP1 wurde mit HindIII und XbaI geschnitten. In den derart behandelten Vektor wurde ein ca. 2,7 kb großes HindIII-XbaI-Fragment aus pFXIII-13, das die F XIIIa-cDNA trägt, ligiert. Die F XIIIa-Transkriptionseinheit auf pSVF13 besitzt keine mRNA-"splice sites".

pZF13 (Fig. 5b): Aus dem Plasmid pFXIII-13 wurde ein ca. 2,7 kb großes HindIII-Fragment isoliert, das die F XIIIa-cDNA enthält. Der entstehende 5'-Überhang wurde durch Auffüllen des Gegenstranges mit DNA Polymerase I (Klenow Fragment) beseitigt. Der Expressionsvektor pZET4 wurde durch Schneiden an der singulären XbaI-Stelle linearisiert. Der entstehende 5'-Überhang wurde ebenfalls durch Auffüllen des Gegenstranges mit DNA-Polymerase I (Klenow-Fragment) beseitigt. Ligation des aufgefüllten Vektors mit dem aufgefüllten F XIIIa-cDNA-Fragment führt zu dem F XIIIa-Expressionsplasmid pZF13. Wie in pSVF13 steht die F XIIIa-cDNA unter der transkriptionellen Kontrolle des SV40 "early"-Promotors, jedoch versehen mit mRNA-"splice sites".

pHSF13 (Fig. 5c): Das Plasmid pSVF13 wurde partiell mit EcoRI verdaut und ein ca. 2,9 kb großes Fragment isoliert, das die F XIIIa-cDNA, gefolgt von der SV40-Polyadenylierungsstelle für frühe Transkripte, trägt. Dieses Fragment wurde in die singuläre EcoRI-Stelle des Plasmids pSP6HS9 "downstream" des "heat shock protein 70"-Promotors ligiert.

### c) DHFR-Expressionsvektoren zur Kotransfektion von CHO (Chinese hamster ovary) dhfr⁻ -Zellen

Zur Kotransfektion mit den beschriebenen F XIIIa-Expressionsvektoren in CHO dhfr⁻-Zellen wurden entweder der DHFR-Vektor pSV2dhfr (Lee et al., a. a. O.) oder das promotorlose DHFR-Plasmid pSVOAdhfr (deutsche Patentanmeldung P 36 24 453.8, siehe Anhang) verwendet. Beide Vektoren tragen die DHFR-cDNA aus der Maus.

### d) G418-Resistenz übertragender Vektor zur Kotransfektion von BHK (baby hamster kidney)-Zellen

Die beschriebenen F XIIIa-Expressionsvektoren wurden mit dem Vektor pRMH140 (Hudziak et al. Cell 31 (1982), 137-146) in BHK-Zellen kotransfiziert.

### e) Expression von F XIIIa in CHO-Zellen

Das Plasmid pZF13 wurde mit dem DHFR-Vektor pSV2dhfr und das Expressionsplasmid PSVF13 zusammen mit dem DHFR-Vektor pSVOAdhfr mit Hilfe der Calciumphosphatpräzipitationsmethode (Graham und van der Eb, Virology 52 (1973), 456-467) in CHO dhfr⁻-Zellen kotransfiziert. Hierzu wurden jeweils 20 µg des F XIIIa-Expressionsplasmids (pZF13 bzw. pSVF13) mit 5 µg der DHFR-Vektoren (pSV2dhfr bzw. pSVOAdhfr) gemischt und kopräzipitiert. Das Kopräzipitat wurde, wie oben beschrieben, zur Transfektion (0,5 x 10⁶ Zellen in einer 25 cm² Kulturflasche) eingesetzt. Nach 3 Tagen wurden die Zellen trypsiniert, in drei 60 mm Petrischalen übertragen und mit Selektionsmedium (ohne Glycin, Hypoxanthin und Thymidin) versetzt. Unter diesen Bedingungen überleben nur Zellen, die stabil mit dem DHFR-Gen transfiziert worden sind. Nach 1-3 Wochen werden Kolonien, bestehend aus transfizierten Zellen, in den Petrischalen sichtbar. Folgende Transfektionsraten konnten hierbei erzielt werden:
pSV2dhfr 5 x 10⁻⁵/Platte
pSVOAdhfr 1 x 10⁻⁵/Platte Einzelne Klone wurden isoliert und in Medium ohne Glycin, Hypoxanthin und Thymidin vermehrt.

Zum Nachweis von F XIIIa in Kulturüberständen und Zelllysaten der einzelnen Klone wurde ein spezifischer ELISA mit einer unteren Detektionsgrenze von ca. 3 ng/ml verwendet.

Kulturüberstände wurden direkt im ELISA eingesetzt. Die Zellysate wurden wie folgt hergestellt:
Konfluente Zellen in 25 cm² Kulturflaschen wurden zweimal in 40 mM Tris-HCl (pH 7,4), 1 mM EDTA, 150 mM NaCl, gewaschen, in 150 µl 0;25 M Tris-HCl (pH 7,8), 5 mM DTT, 2 % Glycerin, 0,2 % Detergenz (Triton X100) aufgenommen und durch dreimaliges Einfrieren und Auftauen lysiert.

Unlösliche Zellbestandteile wurden durch Zentrifugation abgetrennt. Das Lysat wurde nach 1:2,5 Verdünnung in den ELISA eingesetzt. Für die Kombination der Plasmide pZF13/pSV2dhfr konnte durch das beschriebene Nachweisverfahren aus 17 untersuchten Klonen ein F XIIIa exprimierender Klon (CHO 59-5-C7) herausgefunden werden. Nach Kotransfektion mit den Plasmiden pSVF13/pSVOAdhfr konnte bei 12 analysierten Klonen ein weiterer F XIIIa-exprimierender Klon (CHO 60-3-C1) bestimmt werden. Bei beiden positiven Klonen konnte F XIIIa im Medium und im Lysat in gleichen relativen Mengen nachgewiesen werden. Um die Expressionsrate der F XIIIa produzierenden Linien quantitativ zu bestimmen, wurde folgende Standardprozedur durchgeführt:
0,5 x 10⁶ Zellen wurden in 5 ml Medium in 25 cm² Kulturflaschen ausplattiert. Nach 24 Stunden wurde das Medium ausgewechselt (5 ml). Weitere 24 Stunden später wurde das Medium entnommen, die Zellzahl bestimmt und Lysate angefertigt. 6 Bei allen im folgenden angegebenen Expressionsraten (ng/10⁶ Zellen/24 h) lag die Zellzahl/25 cm² Flasche am Ende des Tests bei 1 ± 0,25 x 10⁶ Zellen. Die folgende Übersicht gibt die Expressionsrate der in beschriebener Art und Weise getesteten Basisklone an:

| | extrazellulär (ng/10⁶ Zellen/24 h) | intrazellulär (ng/10⁶ Zellen/24 h) |
|---|---|---|
| CHO 59-5-C7 | 12 | 9 |
| CHO 60-3-C1 | 17 | 13 |

In der SDS-Elektrophorese mit darauffolgendem F XIIIa-spezifischen "Immunoblot" reagiert bei Lysaten und Überständen von Klon CHO 59-5-C7 und Klon CHO 60-3-C1 jeweils eine Bande mit dem Molekulargewicht des aus humaner Plazenta isolierten Proteins.

Der Klon CHO 59-5-C7 wurde zur Genamplifikation steigenden Methotrexat (Mtx)-Konzentrationen ausgesetzt. Beginnend mit einer Konzentration von 10 nM Mtx und einer Adaptionszeit von 4 Passagen wurde die Mtx-Konzentration im Medium auf 50 nM erhöht. In der Standardprozedur wurden folgende Expressionsraten bestimmt:

| Mtx (nM) | extrazellulär (ng/10⁶ Zellen/24 h) | intrazellulär (ng/10⁶ Zellen/24 h) |
|---|---|---|
| 0 | 12 | 9 |
| 10 | 19 | 16 |
| 50 | 38 | 66 |

### f) Expression von F XIIIa in BHK-Zellen

Je 20 µg der F XIIIa-Expressionsplasmide pZF13, pSVF13 und pHSF13 wurden mit 5 µg des für G418-Resistenz kodierenden Plasmids pRMH140 nach der in Beispiel 5e) beschriebenen Calciumphosphatpräzipitationsmethode in BHK-Zellen kotransfiziert. Nach 3 Tagen wurden die Zellen trypsiniert, in drei 60 mm Petrischalen übertragen und mit Selektionsmedium, das 400 µg/ml G418 enthält, versetzt. Nach 12 Tagen waren in jeder Petrischale ca. 200-300 G418-resistente Kolonien gewachsen. Die Gesamtzahl der Klone wurde trypsiniert und als Mischklon (MK) in 25 cm² Kulturflaschen passagiert (5 ml Medium).

Im Falle von pZF13- und pSVF13-transfizierten Zellen wurde F XIIIa bei Erreichen von 80-100 % Konfluenz im Medium und im zugehörigen Lysat (s. Beispiel 5e)) über einen spezifischen ELISA bestimmt. Mischklone, die mit pHSF13 transfiziert worden waren, wurden ebenfalls 80-100 % konfluent mit auf 42°C vorgewärmtem, frischem Medium versetzt und eine Stunde bei 42°C inkubiert. Nach erneutem Mediumwechsel mit auf 37°C temperiertem, frischem Medium wurden die Zellen 24 Stunden bei 37°C gehalten. Medium und Lysate wurden wie oben beschrieben auf ihren Gehalt an F XIIIa untersucht. Die folgende Tabelle gibt für die verschiedenen Mischklone eine Übersicht über die zelluläre Verteilung von F XIIIa.

| | extrazellulär (ng) | intrazellulär (ng) |
|---|---|---|
| BHK-MK1 (pZF13) | 65 | 22 |
| BHK-MK2 (pZF13) | 80 | 20 |
| BHK-MK3 (pSVF13) | 85 | 18 |
| BHK-MK5 (pHSF13) | 170 | 11 |

Die Expressionsraten bezüglich des im Medium vorliegenden F XIIIa wurden für die einzelnen Mischklone nach der im Beispiel 5e) beschriebenen Standardprozedur bestimmt. Bei allen im folgenden angegebenen Expressionsraten (ng/10⁶ Zellen/24 h) lag die Zellzahl/25 cm² Flasche am Ende des Tests bei 4,5 ± 0,5 x 10⁶ Zellen.

| | extrazellulär (ng/10⁶ Zellen/24 h) |
|---|---|
| BHK-MK1 (pZF13) | 3,4 |
| BHK-MK2 (pZF13) | 5,6 |
| BHK-MK5 (pHSF13) | 3,8 |
| BHK-MK6 (pHSF13) | 3,8 |

Da es sich bei den bisher beschriebenen transfizierten BHK-Linien um Mischpopulationen mit nicht produzierenden bzw. sich in den Expressionsraten unterscheidenden Zellen handelt, wurde im folgenden versucht, hochexprimierende genetisch einheitliche Zellinien durch Klonvereinzelung zu isolieren. Zu diesem Zweck wurden Zellen aus dem jeweiligen Mischklon in der Konzentration 1 Zelle/Loch, 2 Zellen/ Loch und 4 Zellen/Loch auf Mikrotiterplatten gegeben. Überstände aus Löchern, in denen nur ein Klon gewachsen war, wurden im F XIIIa-spezifischen ELISA analysiert. Die Klone mit den höchsten Expressionswerten wurden auf 25 cm² Kulturflaschen expandiert und nach der oben beschriebenen Standardprozedur bezüglich ihrer Expressionsraten untersucht. Die folgende Übersicht zeigt die Expressionsrate von Klonen, die aus der Vereinzelung von BHK-MK1 (pZF13) in der beschriebenen Art und Weise hervorgegangen sind.

| | extrazellulär (ng/10⁶ Zellen/24 h) |
|---|---|
| BHK MK1 (pZF13) | 3,4 |
| BHK MK1-A12 ( " ) | 8 |
| BHK MK1-E2 ( " ) | 25 |
| BHK MK1-F12 ( " ) | 14 |
| BHK MKl-Cl ( " ) | 22 |

Es konnte weiterhin am Beispiel der BHK-Zellinie MK1-E2 gezeigt werden, daß von diesen Zellen synthetisierte F XIIIa-Moleküle biologische Aktivität aufweisen. 10⁸ Zellen der Linie BHK MK1-E2 und der verwendeten nicht-transfizierten BHK-Linie (Negativ-Kontrolle) wurden in 1,5 ml 0,25 M Tris-HCl (pH 7,8), das 2 % Glycerin enthält, aufgenommen. Die Zellen wurden durch dreimaliges Einfrieren und Auftauen und anschließende Behandlung in einem Dounce-Homogenisator lysiert. Nach der Abtrennung unlöslicher Bestandteile durch Zentrifugation wurde das Lysat in den biologischen Test (siehe Beispiel 3) eingesetzt. Folgende F XIIIa-Aktivitäten wurden ermittelt:

| | | |
|---|---|---|
| BHK MK1-E2 | 0,06 | Einheiten/10⁸ Zellen |
| BHK (nicht transfiziert) | 0 | " " |

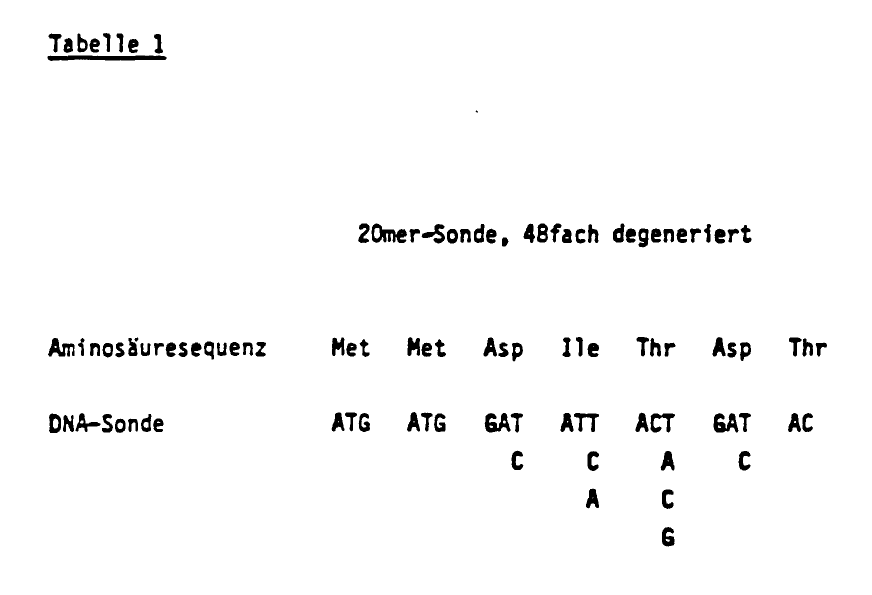

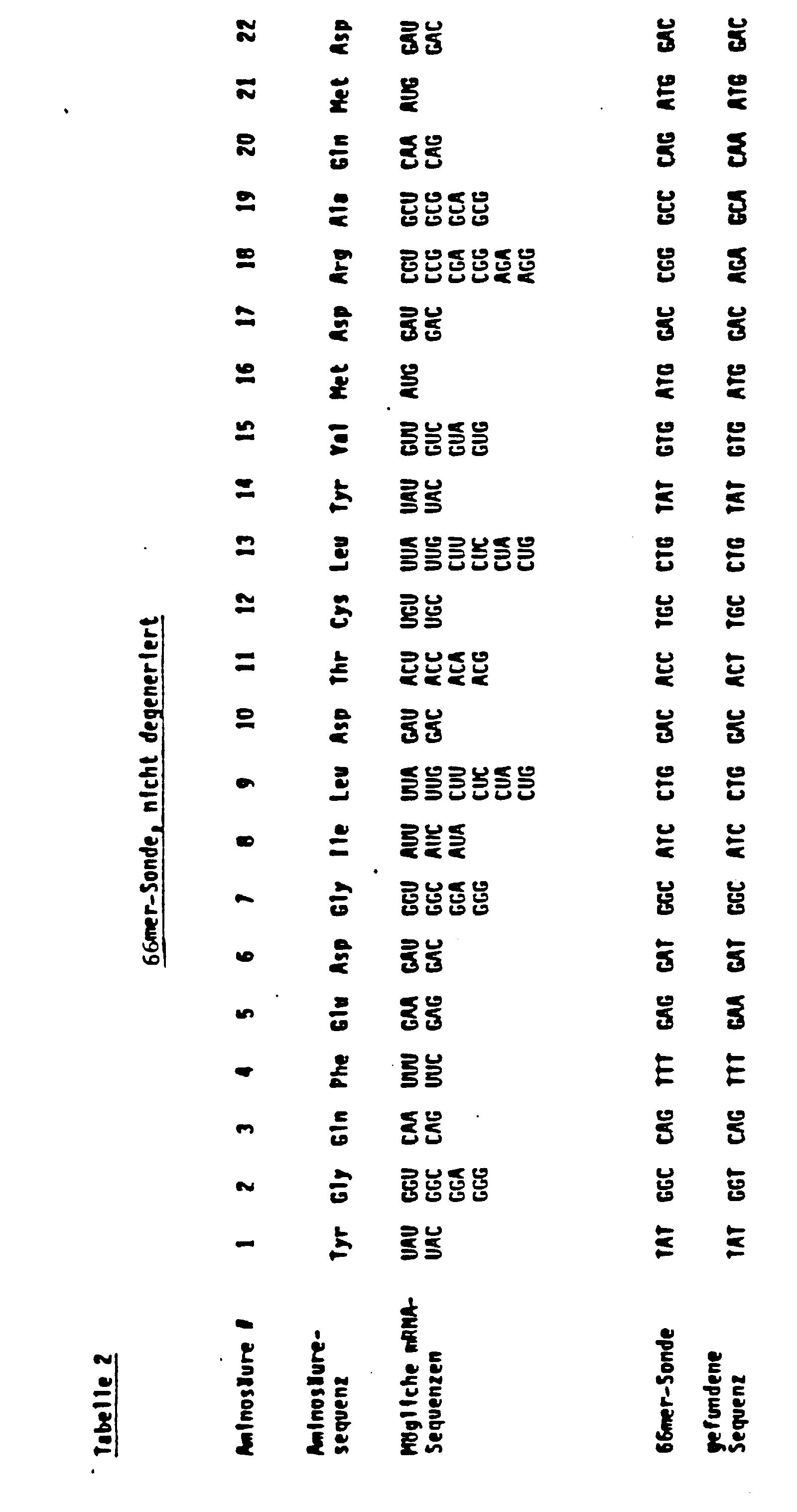

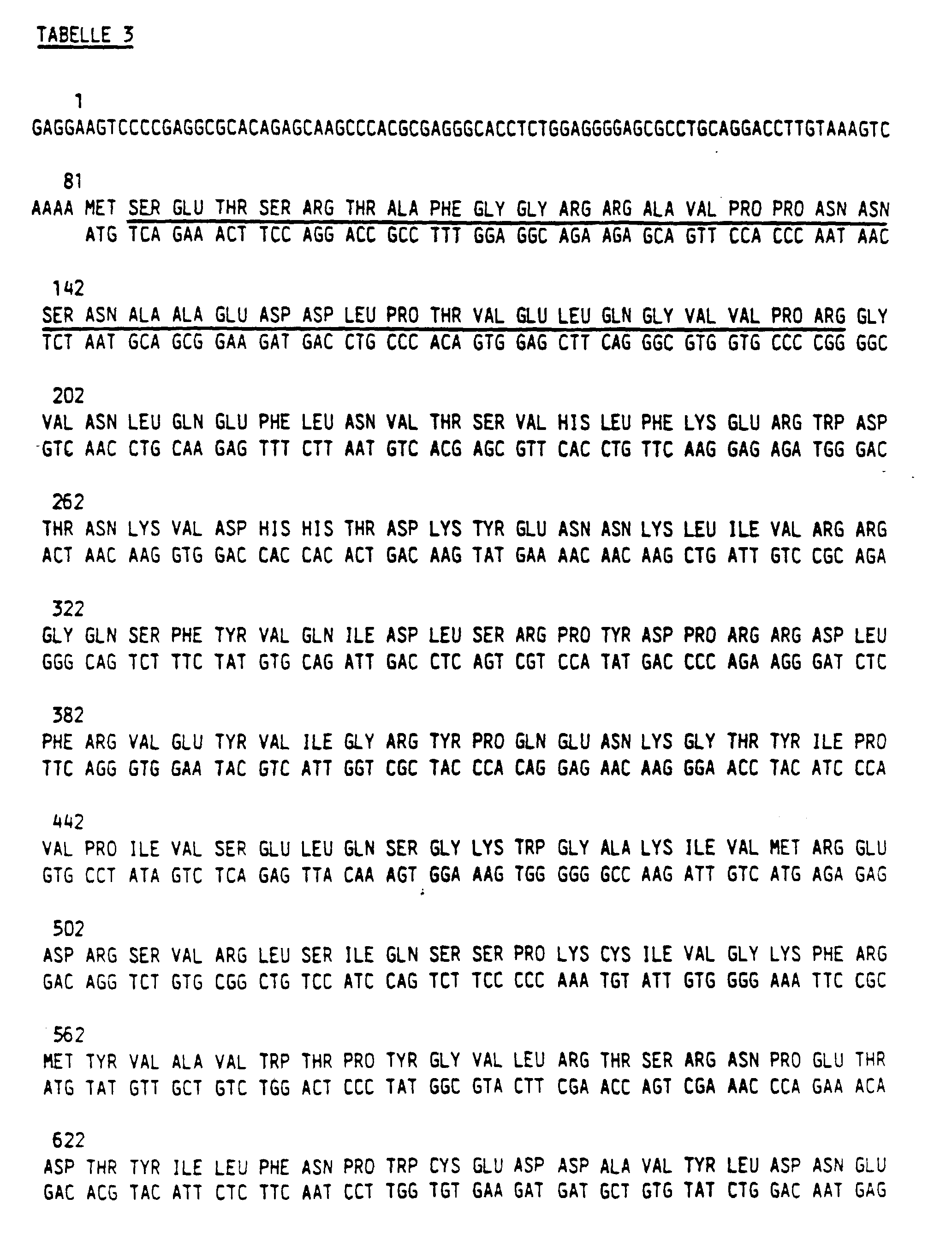

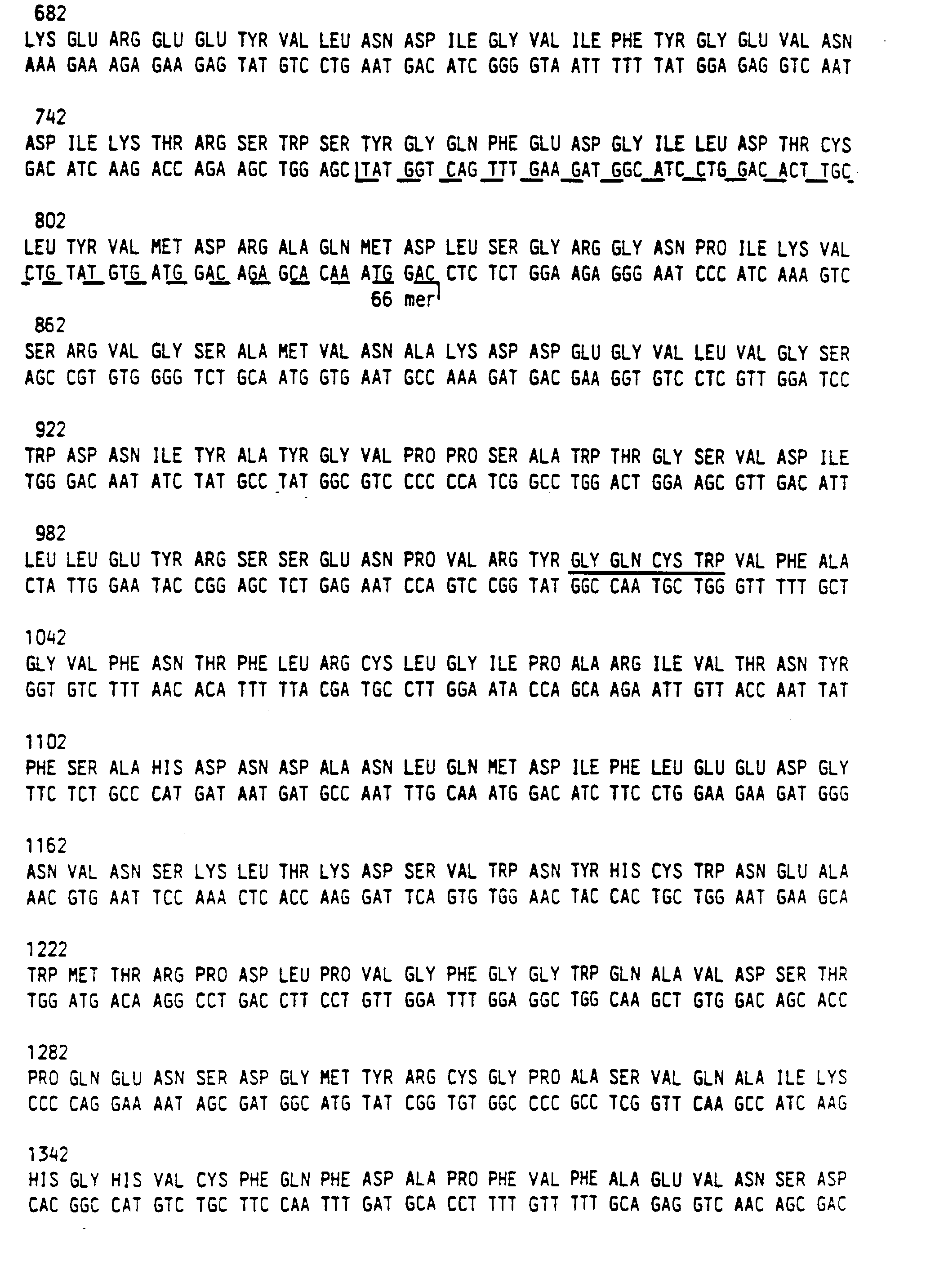

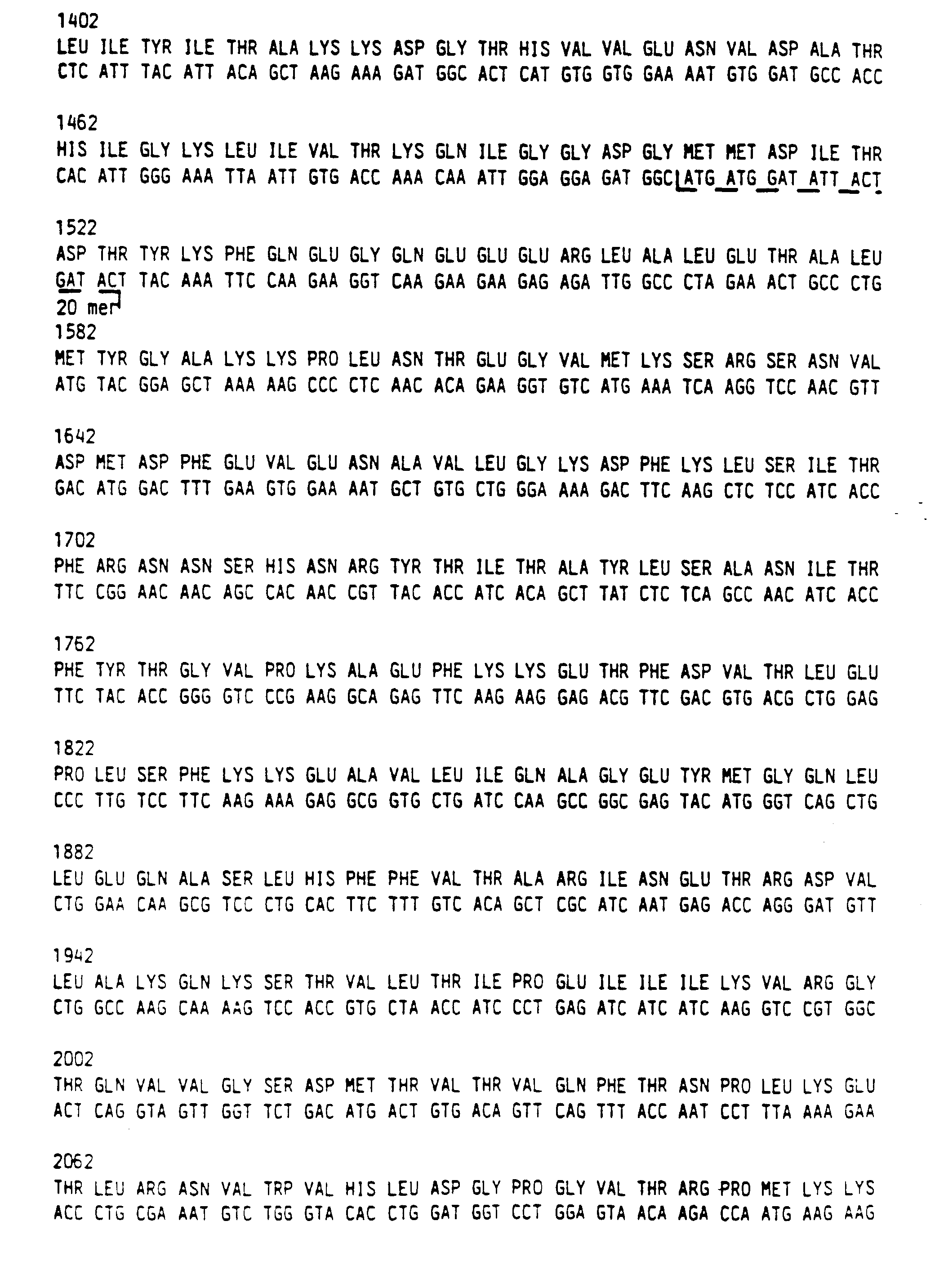

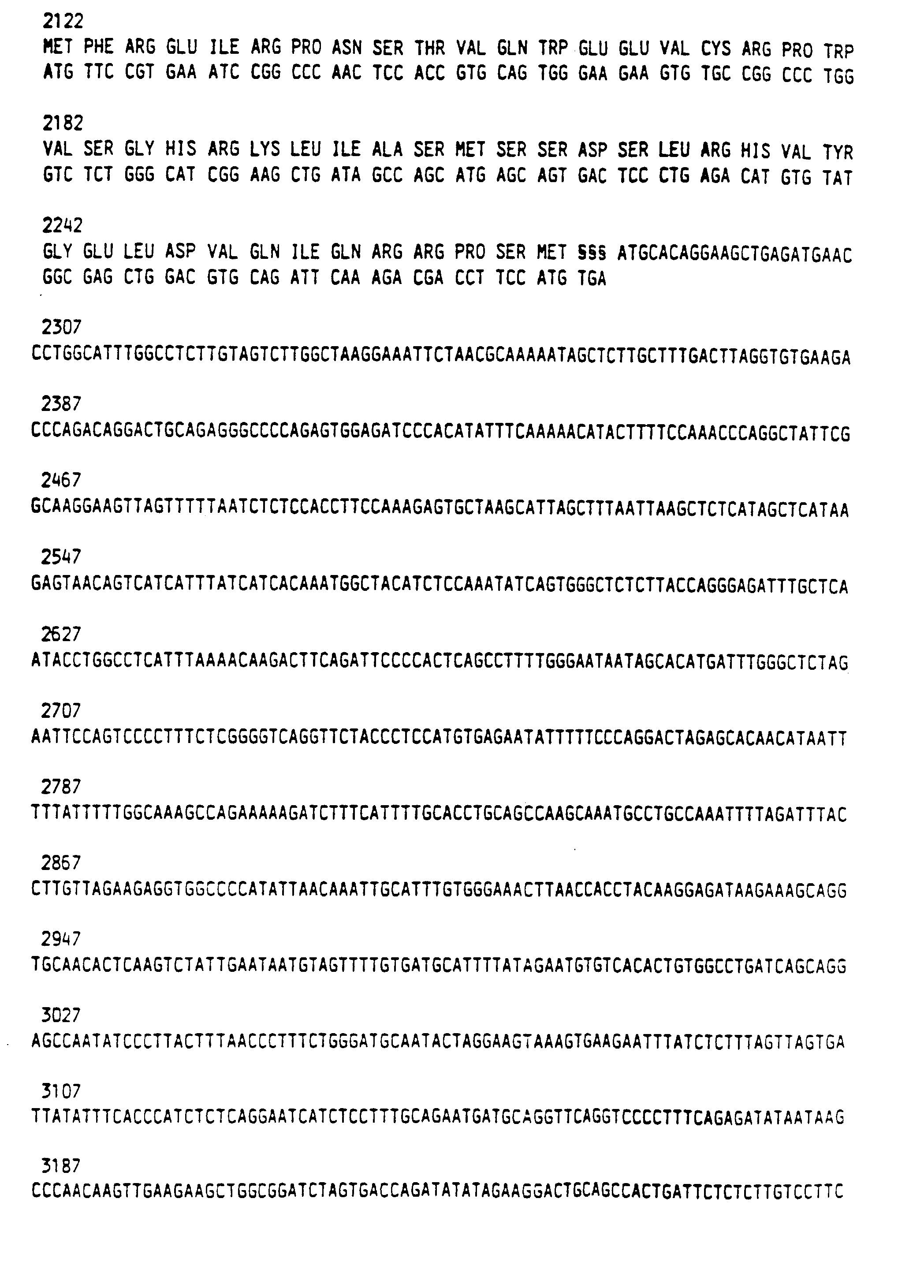

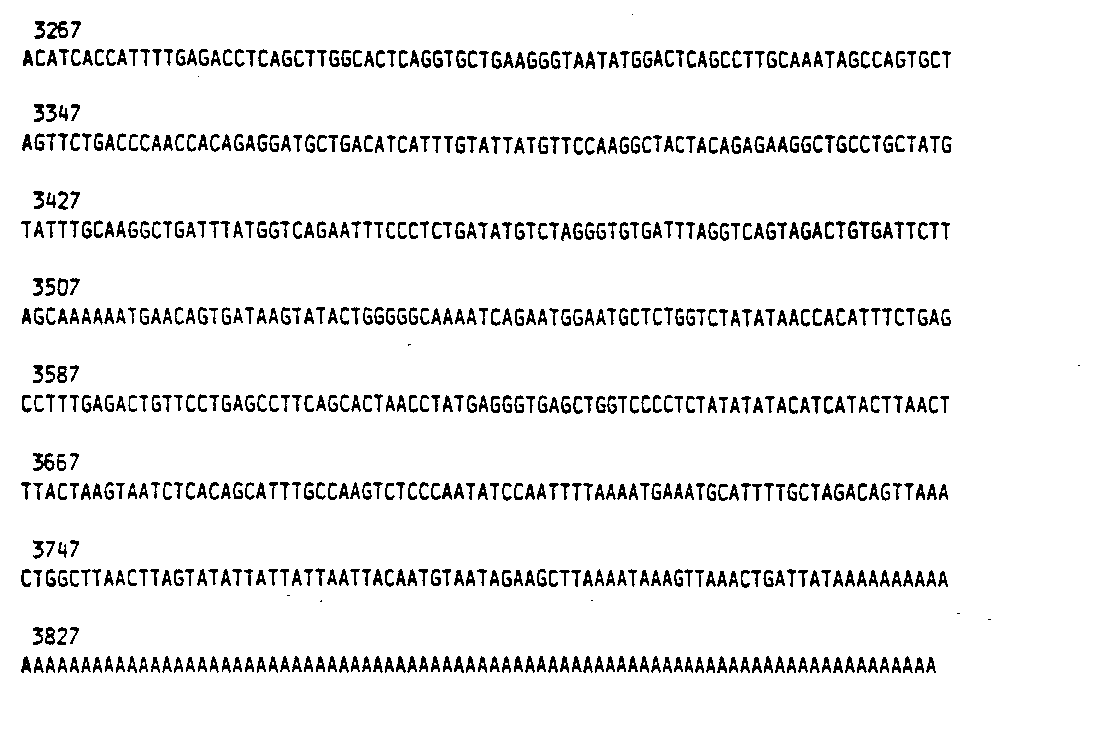

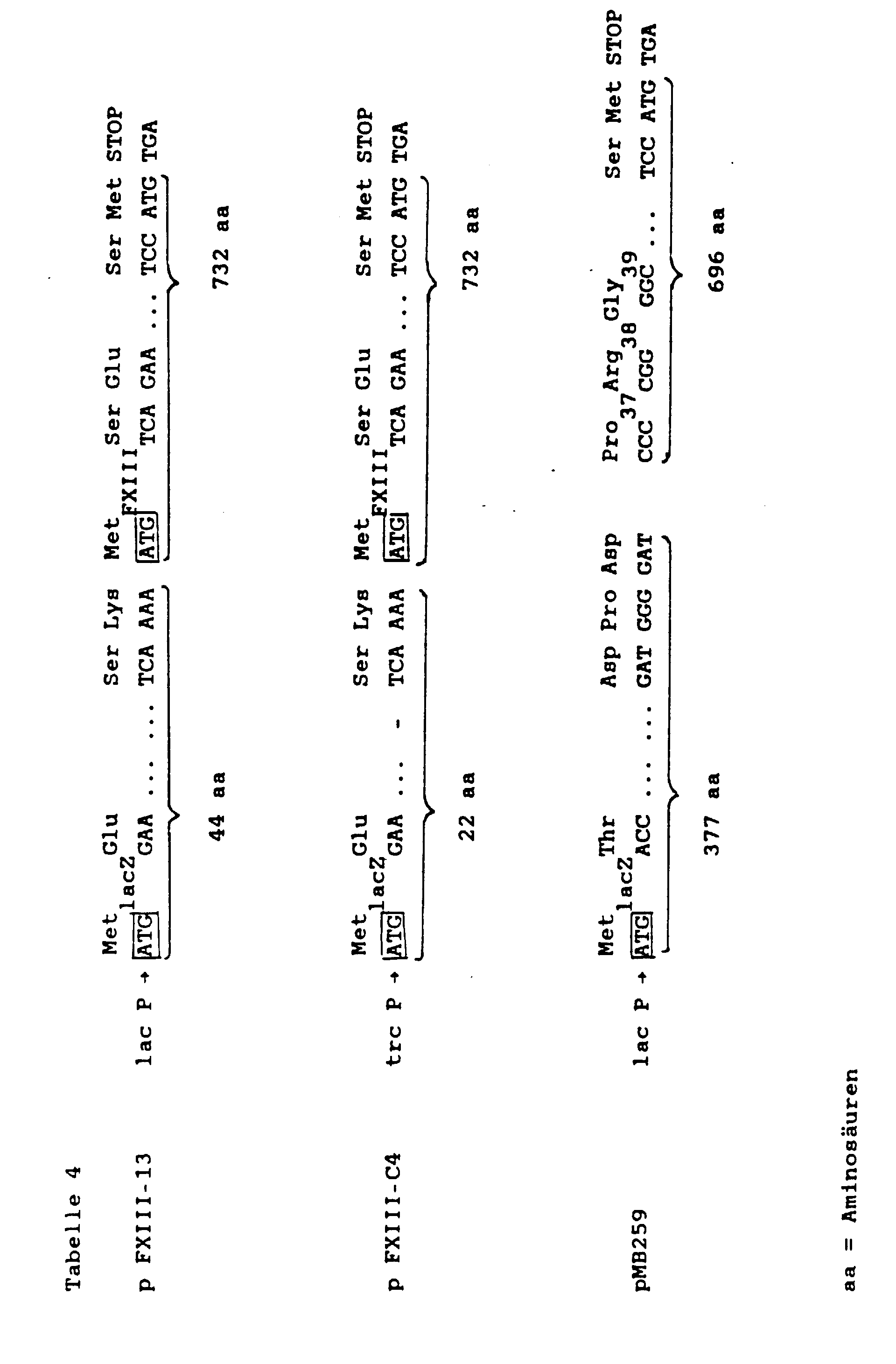

### Anhang

### Beispiel 1 aus der deutschen Patentanmeldung P 36 24 453.8

### a) Konstruktion eines Expressionsvektors für animale Zellen

Das Plasmid pSV2dhfr (Lee et al., a. a. O.) wurde mit HindIII und EcoRI geschnitten und das 2,65 kb Vektorfragment, das den SV40-"early"-Promotor trägt, wurde isoliert. In den derart vorbehandelten Vektor wurde ein 67 bp HindIII-EcoRI-Fragment aus pUC12 STOP (Bröker und Amann, Appl. Microbiol. Biotechnol. 23 (1986) 294-296) ligiert, was zu dem Plasmid pSV2 STOP führt. Auf dem 67 bp-Fragment aus pUC12 STOP befinden sich Translationsstopcodons in allen drei Leserastern. pSV2 STOP wurde mit SacI linearisiert und der entstehende 3'-Überhang mit Hilfe der 3'→5'-Exonukleaseaktivität der DNA-Polymerase I entfernt. Anschließend wurde mit EcoRI verdaut. Nach Ligierung mit einem 133 bp großen EcoRI-HpaI-Fragment aus pBB3 (B. Bourachot et al., EMBO J. 1 (1982) 895-900), das das SV40-Polyadenylierungssignal für frühe Transkripte trägt, konnte der Expressionsvektor pSVA STOP1 erhalten werden.

Die Polyadenylierungsstelle kann auch aus dem Vektor pIG6 (Bourachot et al., a. a. O.) isoliert werden. Genauso kann man aus dem SV40-Genom das 133bp BamHI-HpaI-Fragment isolieren, die BamHI-Schnittstelle auffüllen und einen EcoRI-Linker ansetzen.

pSVA STOP1 trägt somit zwischen dem SV40-"early"-Promotor und dem SV40-Polyadenylierungssignal für frühe Transkripte einen Klonierungspolylinker mit drei singulären Restriktionsstellen (HindIII-SalI-XbaI) und eine Sequenz mit Translationsstops in allen drei Leserastern.

### c) Konstruktion von DHFR-Expressionsvektoren zur Kotransfektion

Ausgangspunkt für die zur Kotransfektion verwendeten DHFR-Vektoren war das Plasmid pMTVdhfr (Lee et al., a. a. O.). pMTVdhfr wurde mit BglII geschnitten und die überstehenden 5'-Enden der DNA mit Hilfe der DNA-Polymerase I (Klenow-Fragment) aufgefüllt. Nach Verdauung mit EcoRI wurde ein 4,47 kb großes Fragment isoliert und mit einem 133 bp EcoRI-HpaI-Fragment aus pBB3 (Bourachot et al., a. a. O.) ligiert. Das neue Plasmid pMTVAdhfr trägt die Maus-DHFR-cDNA, flankiert vom MMTV-LTR und der SV40-Polyadenylierungsstelle für frühe Transkripte.

pSVOAdhfr wurde aus pMTVAdhfr durch Deletion eines 1450 bp großen HindIII-Fragmentes erhalten, das den MMTV-LTR trägt.

Weder pMTVAdhfr noch pSVOAdhfr besitzen m-RNA-"splice sites".

## Patentansprüche (Patentansprüche für folgende(n) Vertragsstaat(en): BE, CH, LI, DE, FR, GB, IT, LU, NL, SE)

1. Hochreines Protein mit Faktor Xllla-Aktivität, enthaltend die Aminosäuresequenz gemäß Tabelle 3.

2. Hochreiner aktivierter Faktor Xllla, enthaltend die Aminosäuresequenz gemäß Tabelle 3 von 39 Gly bis 732 Met.

3. Verfahren zur Herstellung eines hochreinen Proteins mit Faktor Xllla-Aktivität, dadurch gekennzeichnet, daß eine Zelle, welche mit einer DNA, kodierend für die Aminosäruesequenz gemäß Tabelle 3 transformiert wurde, kultiviert und das genannte Protein isoliert wird.

4. Verfahren zur Herstellung eines hochreinen aktivierten Faktor XIIIa, dadurch gekennzeichnet, daß eine Zelle, welche mit einer DNA, kodierend für die Aminosäuresequenz gemäß Tabelle 3 von 39 Gly bis 732 Met mit F Xllla-Aktivität transformiert wurde, kultiviert und das genannte Protein isoliert wird.

## Patentansprüche (Patentansprüche für folgende(n) Vertragsstaat(en): AT, ES)

1. Verfahren zur Herstellung eines hochreinen Proteins mit Faktor Xllla-Aktivität, dadurch gekennzeichnet, daß eine Zelle, welche mit einer DNA, kodierend für die Aminosäuresequenz gemäß Tabelle 3 transformiert wurde, kultiviert und das genannte Protein isoliert wird.

2. Verfahren zur Herstellung eines hochreinen aktivierten Faktor Xllla, dadurch gekennzeichnet, daß eine Zelle, welche mit einer DNA, kodierend für die Aminosäuresequenz gemäß Tabelle 3 von 39 Gly bis 732 Met transformiert wurde, kultiviert und das genannte Protein isoliert wird.

## Claims (Claims for the following Contracting State(s): BE, CH, LI, DE, FR, GB, IT, LU, NL, SE)

1. A high-purity protein having factor XIIIa activity, containing the amino acid sequence shown in Table 3.

2. A high-purity activated factor XIIIa, containing the amino acid sequence shown in Table 3 from 39 Gly to 732 Met.

3. A process for the preparation of a high-purity protein having factor XIIIa activity, which comprises cultivating a cell which has been transformed by means of a DNA encoding the amino acid sequence shown in Table 3, and isolating said protein.

4. A process for the preparation of a high-purity activated factor XIIIa, which comprises cultivating a cell which has been transformed by means of a DNA encoding the amino acid sequence shown in Table 3 from 39 Gly to 732 Met having F XIIIa activity, and isolating said protein.

## Claims (Claims for the following Contracting State(s): AT, ES)

1. A process for the preparation of a high-purity protein having factor XIIIa activity, which comprises cultivating a cell which has been transformed by means of a DNA encoding the amino acid sequence shown in Table 3, and isolating said protein.

2. A process for the preparation of a high-purity activated factor XIIIa, which comprises cultivating a cell which has been transformed by means of a DNA encoding the amino acid sequence shown in Table 3 from 39 Gly to 732 Met, and isolating said protein.

## Revendications (Revendications pour l'(les) Etat(s) contractant(s) suivant(s): BE, CH, LI, DE, FR, GB, IT, LU, NL, SE)

1. Protéine très pure à activité de facteur XIIIa, comportant la séquence d'aminoacides selon le tableau 3.

2. Facteur XIIIa activé très pur, comportant la séquence d'aminoacides selon le tableau 3, de 39 Gly à 732 Met.

3. Procédé pour la production d'une protéine très pure à activité de facteur XIIIa, caractérisé en ce que l'on cultive une cellule qui a été transformée par un ADN codant pour la séquence d'aminoacides selon le tableau 3, et on isole ladite protéine.

4. Procédé pour la production d'un facteur XIIIa activé très pur, caractérisé en ce que l'on cultive une cellule qui a été transformée par un ADN codant pour la séquence d'aminoacides selon le tableau 3, de 39 Gly à 732 Met, à activité de facteur XIIIa, et on isole ladite protéine.

## Revendications (Revendications pour l'(les) Etat(s) contractant(s) suivant(s): AT, ES)

1. Procédé pour la production d'une protéine très pure à activité de facteur XIIIa, caractérisé en ce que l'on cultive une cellule qui a été transformée par un ADN codant pour la séquence d'aminoacides selon le tableau 3, et on isole ladite protéine.

2. Procédé pour la production d'un facteur XIIIa activé très pur, caractérisé en ce que l'on cultive une cellule qui a été transformée par un ADN codant pour la séquence d'aminoacides selon le tableau 3, de 39 Gly à 732 Met, et on isole ladite protéine.
